# EUROPEAN PATENT SPECIFICATION

(11) **EP 2 046 349 B1**
(45) Date of publication and mention of the grant of the patent: **09.09.2015**
(21) Application number: 07784641.8
(22) Date of filing: 12.07.2007
(51) Int. Cl.: A61K 35/28, A61P 17/00, A61P 35/00, A61P 9/10, A61P 31/04, A61P 9/00, A61P 27/02, A61P 7/04, A61P 19/02, A61P 1/00, A61P 17/06, A01K 67/027, C12N 5/0775, A61K 35/12

(54) **TREATMENT OF EXCESSIVE NEOVASCULARIZATION**
BEHANDLUNG VON EXZESSIVER NEOVASKULARISATION
TRAITEMENT DE LA NÉOVASCULARISATION EXCESSIVE

(30) Priority: 12.07.2006 US 830651 P; 12.07.2006 US 830649 P
(43) Date of publication of application: 15.04.2009
(73) Proprietor: Mesoblast, Inc., New York NY 10016 (US)
(72) Inventor: RAKOCZY, Piroska, Elizabeth, Nedlands, Western Australia 6009 (AU)
(74) Representative: Docherty, Robert Charles
(86) International application number: PCT/AU2007/000974
(87) International publication number: WO 2008/006168

(56) References cited:
- WO-A1-98/34485
- WO-A1-2004/084921
- WO-A1-2004/085630
- WO-A1-2006/108229
- WO-A2-01/11011
- WO-A2-02/051980
- WO-A2-2004/010959
- WO-A2-2004/043401
- WO-A2-2004/084950
- WO-A2-2006/091895
- WO-A2-2006/094106
- WO-A2-2006/133052
- WO-A2-2007/035843
- CA-A1- 2 216 439
- OTANI A. ET AL.: 'Bone Marrow-Derived Stem Cells Target Retinal Astrocytes and can Promote or Inhibit Retinal Angiogenesis' NATURE MEDICINE vol. 8, no. 9, 2002, pages 1004 - 1010, XP002988691
- SCHWARTZ Y. AND KORNOWSKI R.: 'Progenitor and Embryonic Stem Cell Transplantation for Myocardial Angiogenesis and Functional Restoration' EUROPEAN HEART JOURNAL vol. 24, 2003, pages 404 - 411, XP009025130
- CABALLERO S. ET AL.: 'The Many Possible Roles of Stem Cells in Age-Related Macular Degeneration' GRAEFE'S ARCHIVE OF CLINICAL AND EXPERIMENTAL OPHTHALMOLOGY vol. 242, 2004, pages 85 - 90, XP008102054
- VAN LAAR J.M. AND TYNDALL A.: 'Adult Stem Cells in the Treatment of Autoimmune Diseases' RHEUMATOLOGY vol. 45, 2006, pages 1187 - 1193, XP008075546
- AL-KHALDI A ET AL: "Postnatal bone marrow stromal cells elicit a potent VEGF-dependent neoangiogenic response in vivo", GENE THERAPY, NATURE PUBLISHING GROUP, GB, vol. 10, 1 January 2003 (2003-01-01), pages 621-629, XP007913740, ISSN: 0969-7128
- GRONTHOS STAN ET AL: "A novel monoclonal antibody (STRO-3) identifies an isoform of tissue nonspecific alkaline phosphatase expressed by multipotent bone marrow stromal stem cells.", STEM CELLS AND DEVELOPMENT DEC 2007, vol. 16, no. 6, December 2007 (2007-12), pages 953-963, ISSN: 1547-3287

## Description

### FIELD OF THE INVENTION

The present invention relates to the use of adult STRO-1^{bright} mesenchymal precursor cells (MPC), or expanded cells thereof, in the manufacture of a medicament for treating or preventing macular degeneration or diabetic retinopathy in a subject, and to adult STRO-1^{brigtht} mesenchymal precursor cells (MPC), or expanded cells thereof, for use in treating or preventing macular degeneration or diabetic retinopathy.

### BACKGROUND OF THE INVENTION

### Angiogenesis

Angiogenesis (or neovascularisation) is the formation and differentiation of new blood vessels. Angiogenesis is generally absent in healthy adult or mature tissue. However, it occurs in the healthy body for healing wounds and for restoring blood flow to tissues after injury or insult. In females, angiogenesis also occurs during the monthly reproductive cycle and during pregnancy. Under these processes, the formation of new blood vessels is strictly regulated.

### Angiogenesis and Disease

In many serious disease states, the body loses control over angiogenesis. Excessive angiogenesis occurs in diseases such as cancer, macular degeneration, diabetic retinopathy, arthritis, and psoriasis. In these conditions, new blood vessels feed diseased tissues, destroy normal tissues, and in the case of cancer, the new vessels allow tumor cells to escape into the circulation and lodge in other organs (tumor metastasis).

The hypothesis that tumor growth is angiogenesis-dependent was first proposed in 1971 (Folkman, 1971). In its simplest terms the hypothesis proposes that expansion of tumor volume beyond a certain phase requires the induction of new capillary blood vessels. For example, pulmonary micrometastases in the early prevascular phase in mice would be undetectable except by high power microscopy on histological sections. Further indirect evidence supporting the concept that tumor growth is angiogenesis dependent is found in U.S. Pat. Nos. 5,639,725, 5,629,327, 5,792,845, 5,733,876, and 5,854,205.

To stimulate angiogenesis, tumors upregulate their production of a variety of angiogenic factors, including the fibroblast growth factors (αFGF and βFGF) (Kandel et al., 1991) and vascular endothelial cell growth factor/vascular permeability factor (VEGF/VPF) and HGF. However, many malignant tumors also generate inhibitors of angiogenesis, including angiostatin protein and thrombospondin. (Chen et al., 1995; Good et al., 1990; O'Reilly et al., 1994). It is postulated that the angiogenic phenotype is the result of a net balance between these positive and negative regulators of neovascularization. (Good et al., 1990; O'Reilly et al., 1994). Several other endogenous inhibitors of angiogenesis have been identified, although not all are associated with the presence of a tumor. These include, platelet factor 4 (Gupta et al., 1995; Maione et al., 1990), interferon-alpha, interferon-inducible protein 10 (Angiolillo et al., 1995; Strieter et al., 1995), which is induced by interleukin-12 and/or interferon-gamma (Voest et al., 1995), gro-beta (Cao et al., 1995), and the 16 kDa N-terminal fragment of prolactin (Clapp et al.,1993).

Neovascularization in the eye is the basis of severe ocular diseases such as age-related macular degeneration (AMD) and Diabetic retinopathy. AMD is the most common cause of legal, irreversible blindness in patients aged 65 and over in the US, Canada, England, Wales, Scotland and Australia. Although the average age of patients when they lose central vision in their first eye is about 65 years, some patients develop evidence of the disease in their fourth or fifth decade of life. Approximately 10% to 15% of patients manifest the exudative (wet) form of the disease. Exudative AMD is characterized by angiogenesis and the formation of pathological neovasculature. The disease is bilateral with accumulating chances of approximately 10% to 15% per annum of developing the blinding disorder in the fellow eye.

Diabetic retinopathy is a complication of diabetes that occurs in approximately 40 to 45 percent of those diagnosed with either Type I or Type II diabetes. Diabetic retinopathy usually effects both eyes and progresses over four stages. The first stage, mild nonproliferative retinopathy, is characterized by microaneuryisms in the eye. Small areas of swelling in the capillaries and small blood vessels of the retina occurs. In the second stage, moderate nonproliferative retinopathy, the blood vessels that supply the retina become blocked. In severe nonproliferative retinopathy, the third stage, the obstructed blood vessels lead to a decrease in the blood supply to the retina, and the retina signals the eye to develop new blood vessels (angiogenesis) to provide the retina with blood supply. In the fourth and most advanced stage, proliferative retinopathy, angiogenesis occurs, but the new blood vessels are abnormal and fragile and grow along the surface of the retina and vitreous gel that fills the eye. When these thin blood vessels rupture or leak blood, severe vision loss or blindness can result.

### Angiogenesis Inhibitors

An example of an angiogenesis inhibitor that specifically inhibits endothelial cell proliferation is angiostatin protein (O'Reilly et al., 1994). Angiostatin protein is an approximately 38 kDa specific inhibitor of endothelial cell proliferation. Angiostatin protein is an internal fragment of plasminogen containing at least three of the five kringles of plasminogen. Angiostatin protein has been shown to reduce tumor weight and to inhibit metastasis in certain tumor models (O'Reilly et al., 1994). Another angiogenesis inhibitor is endostatin protein, which is a carboxy fragment of collagen or XVIII (O'Reilly et al., 1997).

The document by Otani A. et al. (2002), Nature Medicine, vol.8, pp.104-1010 describes that *'intravitreally injected Lin⁻ bone marrow cells (BM) selectively target retinal astrocytes',* and suggests that '*selective targeting with Lin⁻ hematopoietic stem cells (HSC) may be a useful therapeutic approach for the treatment of many ocular diseases'.*

The document WO2004084921 describes induction of neovascularisation by Stro-1^{Bright} cells, in e.g. tumour grafts or in myocardium resulting in *"significant improvement in both global systolic and diastolic parameters of cardiac function"* (p.41 In.9-10).

There is a need for the discovery and development of additional anti-angiogenic agents that may be used alone, or in combination with known angiogenic agents, in order to treat or prevent angiogenesis-related disorders.

### SUMMARY OF THE INVENTION

The present invention provides:
1. Use of adult STRO-1^{bright} mesenchymal precursor cells (MPC), or expanded cells thereof, in the manufacture of a medicament for treating or preventing macular degeneration or diabetic retinopathy in a subject.
2. Use according to aspect, 1, wherein the macular degeneration is dry age-related macular degeneration or wet age-related macular degeneration.
3. Use according to aspect 1 or aspect 2, wherein the stem cells are obtained from bone marrow.
4. Use according to any one of aspects 1 to 3, wherein at least some of the cells are genetically modified.
5. Adult STRO-1^{brigtht} mesenchymal precursor cells (MPC), or expanded cells thereof, for use in treating or preventing macular degeneration or diabetic retinopathy.
6. Adult STRO-1^{brigtht} mesenchymal precursor cells (MPC), or expanded cells thereof, according to aspect 5, wherein the macular degeneration is dry age-related macular degeneration or wet age-related macular degeneration.
7. Adult STRO-1^{brigtht} mesenchymal precursor cells (MPC), or expanded cells thereof, according to aspect 5 or aspect 6, wherein the stem cells are obtained from bone marrow.
8. Adult STRO-1^{brigtht} mesenchymal precursor cells (MPC), or expanded cells thereof, according to any one of aspects 5 to 7, wherein at least some of the cells are genetically modified.

In a preferred embodiment, the stem cells are obtained from bone marrow.

Preferably, the STRO-1^{bright} mesenchymal precursor cells are TNAP⁺, STRO-1⁺, VCAM-1⁺, THY-1⁺, STRO-2⁺, CD45⁺, CD146⁺, 3G5⁺ or any combination thereof.

In a further embodiment, the mesenchymal precursor cells have not been culture expanded and are TNAP⁺.

In a preferred embodiment, the progeny cells are obtained by culturing mesenchymal precursor cells *in vitro.*

In a further embodiment, at least some of the cells are genetically modified.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

### BRIEF DESCRIPTION OF THE ACCOMPANYING DRAWINGS

**Figure 1****. CFP and FA of laser photocoagulated eyes**
   Representative photographs of eyes from each time point show the laser lesions (1a arrows) and the associated fluoroscein leakage (indicated by arrows) at 7 days (1b), 14 days (1c) and 28 days (1d).
**Figure 2****. Mean severity scores of laser lesions at each time point**
   Using the densitometry method the extent of CNV was calculated and statistically analysed. The mean severity score was significantly higher at 14 days (p<0.01) compared to 7 days post laser treatment (asterix). However, while the mean score increased between 14 and 28 days the difference was not significant (p>0.05). This result was characteristic of other breeds of rats using this model.

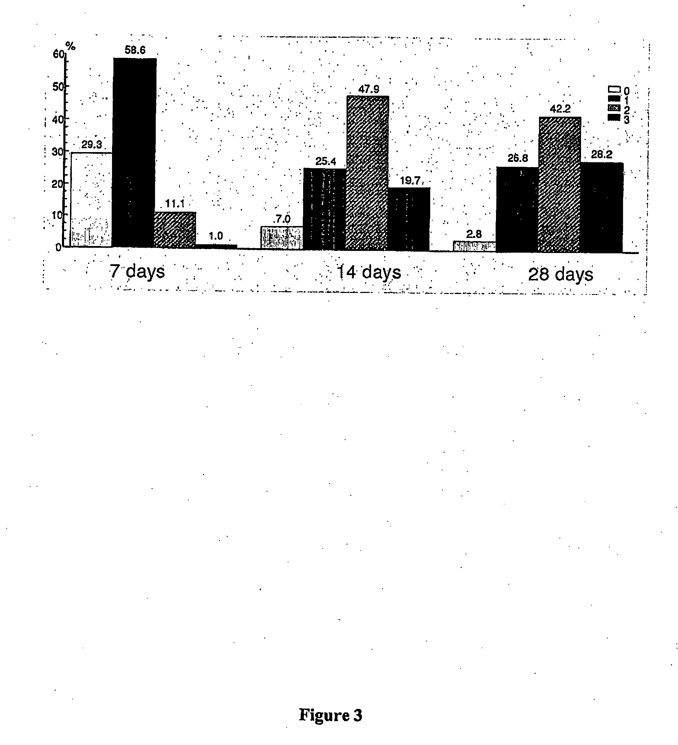

**Figure 3****. Frequency of values of each lesions score**
   The frequency distribution of each lesion score was found to be typical for control values in other rodent breeds using the laser photocoagulation model. This was characterized by zero to mild leakage in the early period and peaking between 14 and 28 days post laser treatment. 0 = zero/no leakage, 1 = mild, 2 = moderate, and 3 = strong leakage.

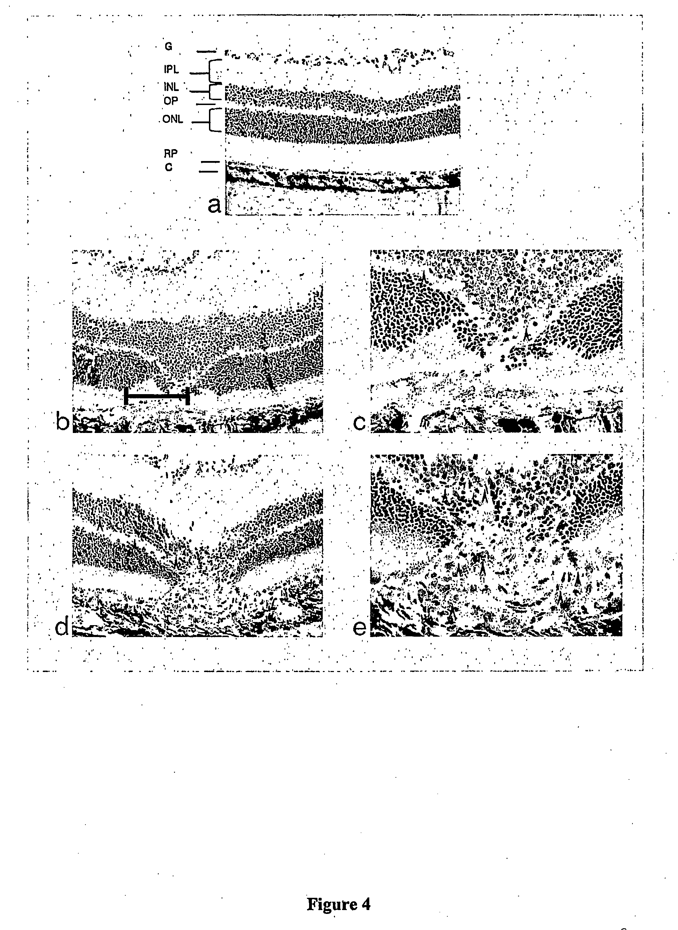

**Figure 4****. H&E stained histological sections of retinal tissue**
   A normal retinal section (a) shows all of the layers intact (GC, ganglion cells; IPL, inner plexiform layer; INL, inner nuclear layer; OPL, outer plexiform layer; ONL outer nuclear layer; RPE, retinal pigment epithelium and CB, choroidal bed). At 20x magnification of an eye 7 days post laser (b), rupture of Bruch's membrane can be seen (bar). Subsequent 40x magnification (c) shows the presence of red blood cells (arrows) within the retinal layers indicating vessel leakage. However, by 28 days the presence of blood cells were far more evident at both 20x (d) and 40x (e) magnification.

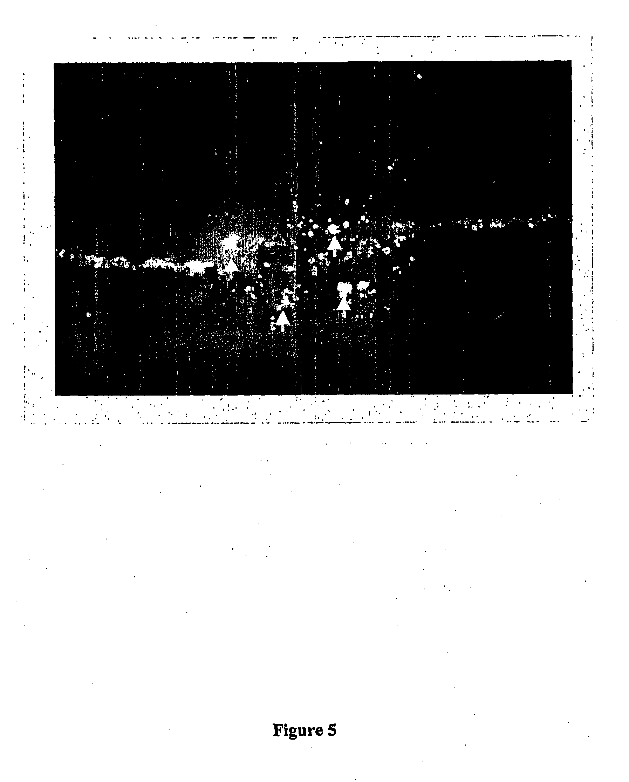

**Figure 5****. Fluorescence microscopy of laser lesion**
   RPE appears yellow and debris from this layer can be seen throughout the lesion site. No macrophages are evident which was confirmed by immunohistochemistry using the CD68 antibody.

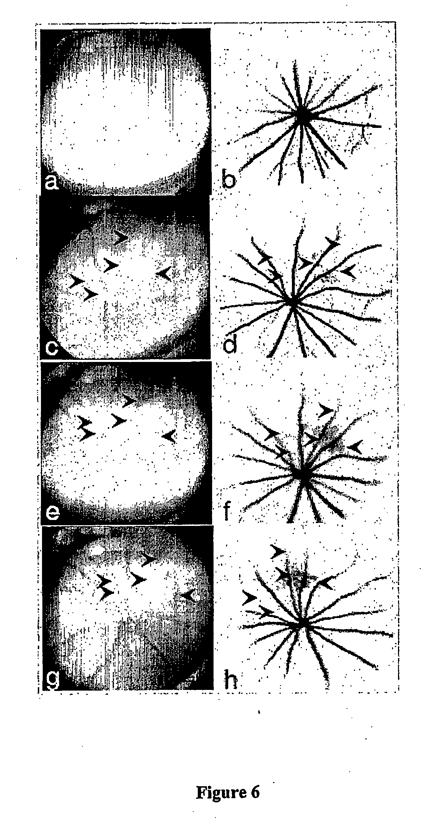

**Figure 6****. CFP and FAs of treated eyes**
   A control, unlasered eye (a and b) was used for comparison. Eyes were evaluated at 7 days (c and d), 14 days (e and f) and 28 days (g and h) for the appearance of the lesions (arrows) using CFP (c, e and g) and FA (d, f and h).

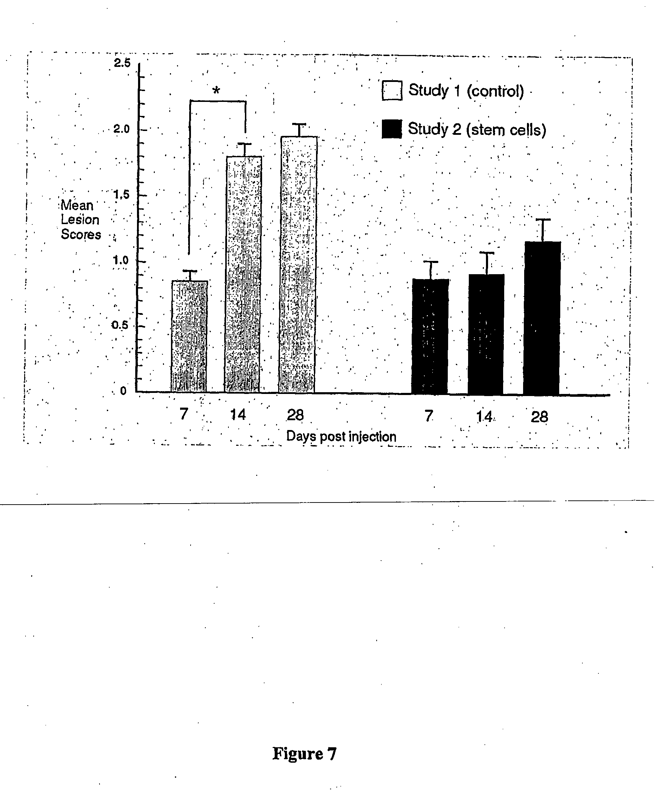

**Figure 7****. Mean leakage scores**
   No significant difference in scores between the control and the study group at day 7 was calculated. Compared to day 7, fluoroscein leakage due to CNV development did not increase significantly at day 14 or 28, which were significantly lower than the control scores at the comparative time points.

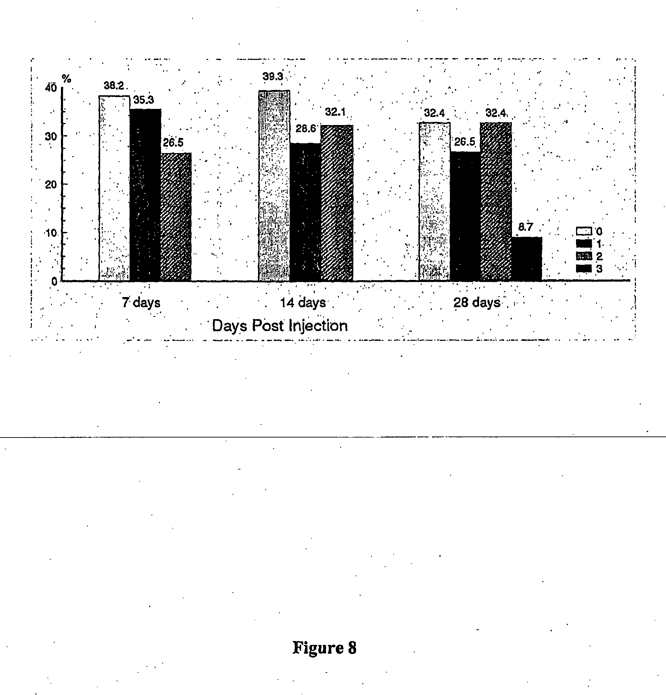

**Figure 8****. Frequency values of each lesions score**
   The frequency distribution of each lesion score was found to be typical for control values in other rodent breeds using the laser photocoagulation model. This was characterized by zero to mild leakage in the early period and peaking between 14 and 28 days post laser treatment. 0 = zero/no leakage, 1 = mild, 2 = moderate, and 3 = strong leakage.

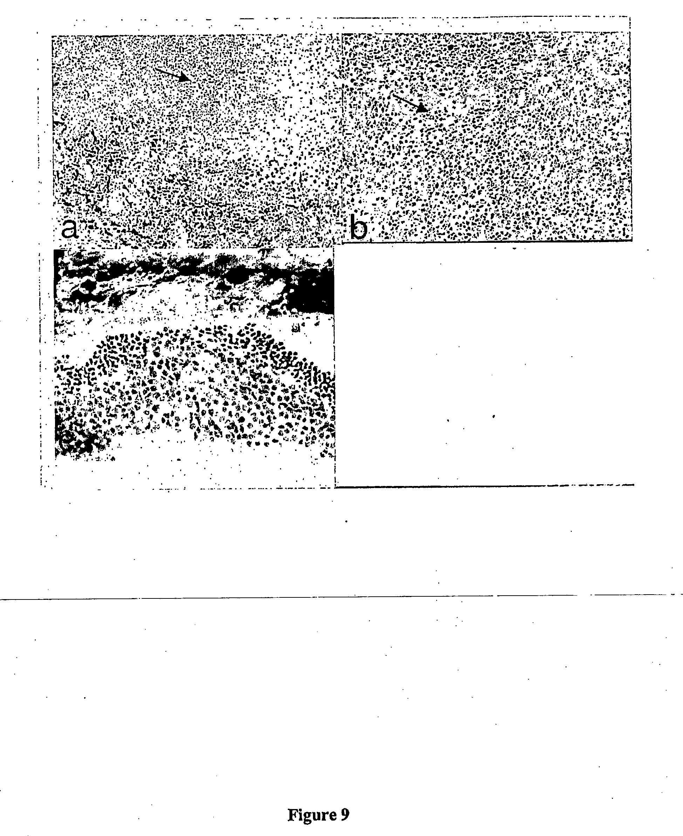

**Figure 9****. Histological sections of eyes**
   Large numbers of macrophages were present in eyes showing signs of endopthalmitus (arrow, 10× a, 20× b). In non-affected eyes the retinal appeared normal with a lack of vascular development in the lesion site (c).

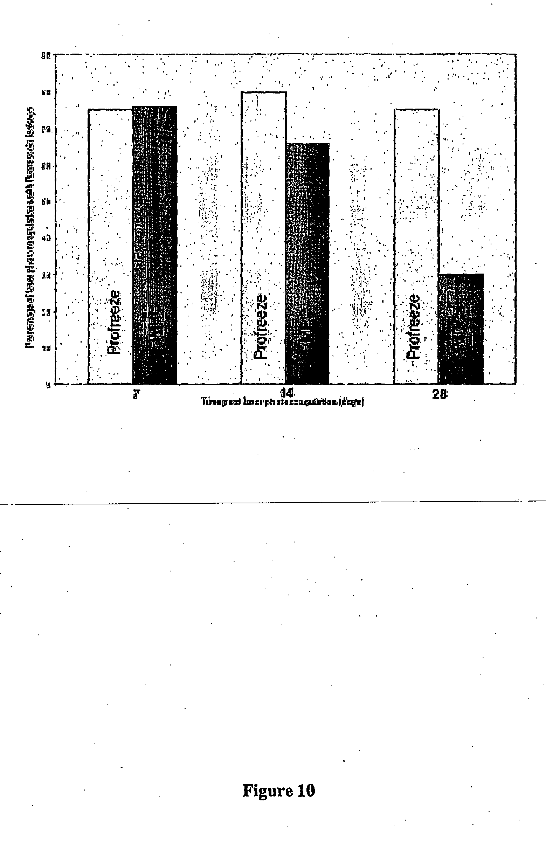

**Figure 10****. Comparison of percentage of laser photocoagulations with fluorescein leakage**
   Graph showing percentage of laser photocoagulations with fluorescein leakage in Profreeze- and cell-injected eyes at different times post-injection.

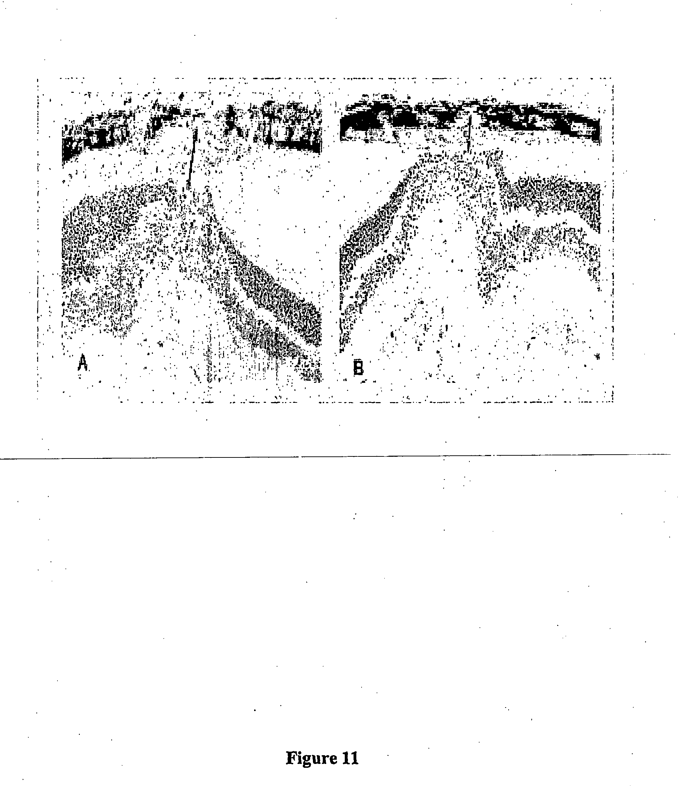

**Figure 11****. Light microscopy of H&E stained paraffin embedded sections of eyes**
   Light microscopy of H&E stained paraffin embedded sections of eyes injected with Profreeze (A) and HMPCs (B). Arrows mark thickness of choroidal neovascular membrane.

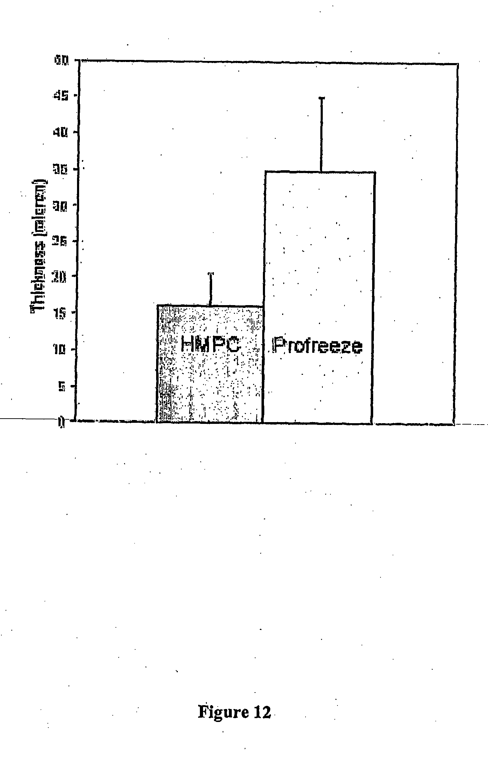

**Figure 12****. Comparison of choroidal neovascular membrane thickness**
   Graph showing the average thickness of 10 choroidal neovascular membranes in cell- and Profreeze-injected eyes.

### DETAILED DESCRIPTION OF THE INVENTION

The invention in its broadest sense is as defined in the independent claims.

### General Techniques

Unless specifically defined otherwise, all technical and scientific terms used herein shall be taken to have the same meaning as commonly understood by one of ordinary skill in the art (e.g., in stem cell biology, cell culture, molecular genetics, immunology, immunohistochemistry, protein chemistry, and biochemistry).

Unless otherwise indicated, the recombinant protein, cell culture, and immunological techniques utilized in the present disclosure are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J.E. Coligan et al. (editors) Current Protocols in Immunology, John Wiley & Sons (including all updates until present).

### Angiogenesis-Related Diseases, and Treatment or Prevention Thereof

As used herein, the term "angiogenesis" is defined as a process of tissue vascularization that involves the growth of new and/or developing blood vessels into a tissue, and is also referred to as neo-vascularization. The process can proceed in one of three ways: the vessels can sprout from pre-existing vessels, *de novo* development of vessels can arise from precursor cells (vasculogenesis), and/or existing small vessels can enlarge in diameter.

As used herein, an "angiogenesis-related disease" is any condition characterized by excessive and/or abnormal neo-vascularization.

Angiogenesis-related diseases include, but are not limited to, angiogenesis-dependent cancer, including, for example, solid tumors, blood bom tumors such as leukemias, and tumor metastases; benign tumors, for example hemangiomas, acoustic neuromas, neurofibromas, trachomas, and pyogenic granulomas; rheumatoid arthritis; psoriasis; ocular angiogenic diseases, for example, diabetic retinopathy, retinopathy of prematurity, macular degeneration including dry age-related macular degeneration and wet age-related macular degeneration, corneal graft rejection, neovascular glaucoma, retrolental fibroplasia, rubeosis; Osler-Webber Syndrome; myocardial angiogenesis blindness; plaque neovascularization; telangiectasia; hemophiliac joints; angiofibroma; and wound granulation. Diseases that have angiogenesis as a pathologic consequence such as cat scratch disease (*Rochele minalia quintosa*) and ulcers (*Helicobacter pylorii*) are also angiogenesis-related.

As used herein, an "ocular angiogenesis disease" is any condition of the eye characterized by excessive and/or abnormal neo-vascularization.

The methods of the disclosure may be combined with other therapies for treating or preventing an angiogenesis-related disease. The nature of these other therapies will depend on the particular angiogenesis-related disease. For example, adult STRO-1^{bright} mesenchymal precursor cells or expanded cells thereof, for use in the treatment or prevention of macular degeneration may be combined with antioxidant and/or zinc supplements, with macugen (Pegaptanib), as defined in US 6,942,655, and/or with laser treatment.

As used herein, the term "subject" (also referred to herein as a "patient") includes warm-blooded animals, preferably mammals, including humans. In a preferred embodiment, the subject is a primate. In an even more preferred embodiment, the subject is a human.

As used herein the terms "treating", "treat" or "treatment" include administering a therapeutically effective amount of cells as defined herein sufficient to reduce or eliminate at least one symptom of an angiogenesis-related disease.

As used herein the terms "preventing", "prevent" or "prevention" include administering a therapeutically effective amount of cells as defined herein sufficient to stop or hinder the development of at least one symptom of an angiogenesis-related disease.

### Stem Cells and Progeny Thereof

As used herein, the term "stem cell" refers to self-renewing cells that are capable of giving rise to phenotypically and genotypically identical daughters as well as at least one other final cell type (e.g., terminally differentiated cells). The term "stem cells" includes totipotential, pluripotential and multipotential cells, as well as progenitor and/or precursor cells derived from the differentiation thereof.

As used herein, the term "totipotent cell" or "totipotential cell" refers to a cell that is able to form a complete embryo (e.g., a blastocyst).

As used herein, the term "pluripotent cell" or "pluripotential cell" refers to a cell that has complete differentiation versatility, i.e., the capacity to grow into any of the mammalian body's approximately 260 cell types. A pluripotent cell can be self-renewing, and can remain dormant or quiescent within a tissue.

By "multipotential cell" or "multipotent cell" we mean a cell which is capable of giving rise to any of several mature cell types. As used herein, this phrase designates adult stem cells and progenitor cells, such as mesenchymal precursor cells (MPC) and multipotential progeny of these cells. Unlike a pluripotent cell, a multipotent cell does not have the capacity to form all of the cell types.

As used herein, the term "progenitor cell" refers to a cell that is committed to differentiate into a specific type of cell or to form a specific type of tissue. Mesenchymal precursor cells (MPCs) are cells found in bone marrow, blood, dental pulp cells, adipose tissue, skin, spleen, pancreas, brain, kidney, liver, heart, retina, brain, hair follicles, intestine, lung, lymph node, thymus, bone, ligament, tendon, skeletal muscle, dermis, and periosteum; and are capable of differentiating into different germ lines such as mesoderm, endoderm and ectoderm. Thus, MPCs are capable of differentiating into a large number of cell types including, but not limited to, adipose, osseous, cartilaginous, elastic, muscular, and fibrous connective tissues. The specific lineage-commitment and differentiation pathway which these cells enter depends upon various influences from mechanical influences and/or endogenous bioactive factors, such as growth factors, cytokines, and/or local microenvironmental conditions established by host tissues. Mesenchymal precursor cells are thus non-hematopoietic progenitor cells which divide to yield daughter cells that are either stem cells or are precursor cells which in time will irreversibly differentiate to yield a phenotypic cell.

In a preferred embodiment, cells used in the invention are enriched from a sample obtained from a subject. The terms 'enriched', 'enrichment' or variations thereof are used herein to describe a population of cells in which the proportion of one particular cell type or the proportion of a number of particular cell types is increased when compared with the untreated population.

In a preferred embodiment, the STRO-1^{bright} MPC used in the present invention are TNAP⁺, STRO-1⁺, VCAM-1⁺, THY-1⁺, STRO-2⁺, CD45⁺, CD146⁺, 3G5⁺ or any combination thereof. Preferably, the STRO-1^{bright} cells are additionally one or more of VCAM-1⁺, THY-1⁺, STRO-2⁺ and/or CD146⁺.

In one embodiment, the mesenchymal precursor cells are perivascular mesenchymal precursor cells as defined in WO 2004/85630.

When we refer to a cell as being "positive" for a given marker it may be either a low (lo or dim) or a high (bright, bri) expresser of that marker depending on the degree to which the marker is present on the cell surface, where the terms relate to intensity of fluorescence or other colour used in the colour sorting process of the cells. The distinction of lo (or dim or dull) and bri will be understood in the context of the marker used on a particular cell population being sorted. When we refer herein to a cell as being "negative" for a given marker, it does not mean that the marker is not expressed at all by that cell. It means that the marker is expressed at a relatively very low level by that cell, and that it generates a very low signal when detectably labelled. The term "bright", when used herein, refers to a marker on a cell surface that generates a relatively high signal when detectably labelled. Whilst not wishing to be limited by theory, it is proposed that "bright" cells express more of the target marker protein (for example the antigen recognised by STRO-1) than other cells in the sample. For instance, STRO-1^{bri} cells produce a greater fluorescent signal, when labelled with a FITC-conjugated STRO-1 antibody as determined by FACS analysis, than non-bright cells (STRO-1^{dull/dim}). Preferably, "bright" cells constitute at least about 0.1% of the most brightly labelled bone marrow mononuclear cells contained in the starting sample. In other embodiments, "bright" cells constitute at least about 0.1%, at least about 0.5%, at least about 1%, at least about 1.5%, or at least about 2%, of the most brightly labelled bone marrow mononuclear cells contained in the starting sample. In a preferred embodiment, STRO-1^{bright} cells have 2 log magnitude higher expression of STRO-1 surface expression. This is calculated relative to "background", namely cells that are STRO-1⁻. By comparison, STRO-1^{dim} and/or STRO-1^{intermediate} cells have less than 2 log magnitude higher expression of STRO-1 surface expression, typically about 1 log or less than "background".

When used herein the term "TNAP" is intended to encompass all isoforms of tissue non-specific alkaline phosphatase. For example, the term encompasses the liver isoform (LAP), the bone isoform (BAP) and the kidney isoform (KAP). In a preferred embodiment, the TNAP is BAP. In a particularly preferred embodiment, TNAP as used herein refers to a molecule which can bind the STRO-3 antibody produced by the hybridoma cell line deposited with ATCC on 19 December 2005 under the provisions of the Budapest Treaty under deposit accession number PTA-7282.

Furthermore, in a preferred embodiment, the cells are capable of giving rise to clonogenic CFU-F.

It is preferred that a significant proportion of the multipotential cells are capable of differentiation into at least two different germ lines. Non-limiting examples of the lineages to which the multipotential cells may be committed include bone precursor cells; hepatocyte progenitors, which are multipotent for bile duct epithelial cells and hepatocytes; neural restricted cells, which can generate glial cell precursors that progress to oligodendrocytes and astrocytes; neuronal precursors that progress to neurons; precursors for cardiac muscle and cardiomyocytes, glucose-responsive insulin secreting pancreatic beta cell lines. Other lineages include, but are not limited to, odontoblasts, dentin-producing cells and chondrocytes, and precursor cells of the following: retinal pigment epithelial cells, fibroblasts, skin cells such as keratinocytes, dendritic cells, hair follicle cells, renal duct epithelial cells, smooth and skeletal muscle cells, testicular progenitors, vascular endothelial cells, tendon, ligament, cartilage, adipocyte, fibroblast, marrow stroma, cardiac muscle, smooth muscle, skeletal muscle, pericyte, vascular, epithelial, glial, neuronal, astrocyte and oligodendrocyte cells.

In an embodiment, the stem cells, and progeny thereof, are capable of differentiation to pericytes.

In another embodiment, the "multipotential cells" are not capable of giving rise, upon culturing, to hematopoietic cells.

Stem cells useful for the methods of the invention are derived from adult tissue. The term "adult" is used in its broadest sense to include a postnatal subject. In a preferred embodiment, the term "adult" refers to a subject that is postpubertal. The term, "adult" as used herein can also include cord blood taken from a female.

The present invention also relates to use of expanded cells which are produced from the *in vitro* culture of the stem cells described herein. Expanded cells used in or for use according to the invention may have a wide variety of phenotypes depending on the culture conditions (including the number and/or type of stimulatory factors in the culture medium), the number of passages and the like. In certain embodiments, the progeny cells are obtained after about 2, about 3, about 4, about 5, about 6, about 7, about 8, about 9, or about 10 passages from the parental population. However, the progeny cells may be obtained after any number of passages from the parental population.

The progeny cells may be obtained by culturing in any suitable medium. The term "medium", as used in reference to a cell culture, includes the components of the environment surrounding the cells. Media may be solid, liquid, gaseous or a mixture of phases and materials. Media include liquid growth media as well as liquid media that do not sustain cell growth. Media also include gelatinous media such as agar, agarose, gelatin and collagen matrices. The term "medium" also refers to material that is intended for use in a cell culture, even if it has not yet been contacted with cells. In other words, a nutrient rich liquid prepared for bacterial culture is a medium. Similarly, a powder mixture that when mixed with water or other liquid becomes suitable for cell culture, may be termed a "powdered medium".

In an embodiment, progeny cells used in or for use according to the invention are obtained by isolating TNAP+ cells from bone marrow using magnetic beads labelled with the STRO-3 antibody, and plated in α-MEM supplemented with 20% fetal calf serum, 2mM L-glutamine and 100µm L-ascorbate-2-phosphate as previously described (see Gronthos et al. (1995) for further details regarding culturing conditions).

In one embodiment, such expanded cells (at least after 5 passages) can be TNAP-, CC9⁺, HLA class I⁺, HLA class II⁻, CD14⁻, CD19⁻, CD3⁻, CD11a-c⁻, CD31⁻, CD86⁻and/or CD80⁻. However, it is possible that under different culturing conditions to those described herein that the expression of different markers may vary. Also, whilst cells of these phenotypes may predominate in the expanded cell population it does not mean that there is not a minor proportion of the cells that do not have this phenotype(s) (for example, a small percentage of the expanded cells may be CC9-). In one preferred embodiment, expanded cells used in or for use according to the invention still have the capacity to differentiate into different cell types.

In one embodiment, an expanded cell population used in or for use according to the invention comprises cells wherein at least 25%, more preferably at least 50%, of the cells are CC9+.

In another embodiment, an expanded cell population used in or for use according to the invention comprises cells wherein at least 40%, more preferably at least 45%, of the cells are STRO-1+.

Progeny cells may express markers selected from the group consisting of LFA-3, THY-1, VCAM-1, ICAM-1, PECAM-1, P-selectin, L-selectin, 3G5, CD49a/CD49b/CD29, CD49c/CD29, CD49d/CD29, CD29, CD18, CD61, integrin beta, 6-19, thrombomodulin, CD10, CD13, SCF, PDGF-R, EGF-R, IGF1-R, NGF-R, FGF-R, Leptin-R, (STRO-2 = Leptin-R), RANKL, STRO-1^{bright} and CD 146 or any combination of these markers.

In one embodiment, the expanded cells are Multipotential Expanded MPC Progeny (MEMPs) as defined in WO 2006/032092. Methods for preparing enriched populations of MPC from which progeny may be derived are described in WO 01/04268 and WO 2004/085630. In an *in vitro* context MPCs will rarely be present as an absolutely pure preparation and will generally be present with other cells that are tissue specific committed cells (TSCCs). WO 01/04268 refers to harvesting such cells from bone marrow at purity levels of about 0.1% to 90%. The population comprising MPC from which progeny are derived may be directly harvested from a tissue source, or alternatively it may be a population that has already been expanded *ex vivo.*

For example, the progeny may be obtained from a harvested, unexpanded, population of substantially purified MPC, comprising at least about 0.1, 1,5, 10, 20, 30, 40, 50, 60, 70, 80 or 95% of total cells of the population in which they are present. This level may be achieved, for example, by selecting for cells that are positive for at least one marker selected from the group consisting of TNAP, STRO-1^{bright}, 3G5⁺, VCAM-1, THY-1, CD146 and STRO-2.

The MPC starting population may be derived from any one or more tissue types set out in WO 01/04268 or WO 2004/085630, namely bone marrow, dental pulp cells, adipose tissue and skin, or perhaps more broadly from adipose tissue, teeth, dental pulp, skin, liver, kidney, heart, retina, brain, hair follicles, intestine, lung, spleen, lymph node, thymus, pancreas, bone, ligament, bone marrow, tendon and skeletal muscle.

MEMPS can be distinguished from freshly harvested MPCs in that they are positive for the marker STRO-1^{bri} and negative for the marker Alkaline phosphatase (ALP). In contrast, freshly isolated MPCs are positive for both STRO-1^{bri} and ALP. In a preferred embodiment of the present invention, at least 15%, 20%, 30%, 40%, 50%, 60%, 70%, 80%, 90% or 95% of the celles used in or for use according to the invention have the phenotype STRO-1^{bri}, ALP⁻. In a further preferred embodiment the MEMPS are positive tor one or more of the markers Ki67, CD44 and/or CD49c/CD29, VLA-3, α3β1. In yet a further preferred embodiment the MEMPs do not exhibit TERT activity and/or are negative for the marker CD18.

In one embodiment, the cells are from a patient with an angiogenesis related disease, cultured *in vitro* using standard techniques and for administration to a patient as an autologous or allogeneic transplant. In an alternative embodiment, cells of one or more of the established human cell lines are used. In another useful embodiment of the invention, cells of a non-human animal (or if the patient is not a human, from another species) are used.

The invention can be practised using cells from any non-human animal species, including but not limited to non-human primate cells, ungulate, canine, feline, lagomorph, rodent, avian, and fish cells. Primate cells with which the invention may be performed include but are not limited to cells of chimpanzees, baboons, cynomolgus monkeys, and any other New or Old World monkeys. Ungulate cells with which the invention may be performed include but are not limited to cells of bovines, porcines, ovines, caprines, equines, buffalo and bison. Rodent cells with which the invention may be performed include but are not limited to mouse, rat, guinea pig, hamster and gerbil cells. Examples of lagomorph species with which the invention may be performed include domesticated rabbits, jack rabbits, hares, cottontails, snowshoe rabbits, and pikas. Chickens (*Gallus gallus*) are an example of an avian species with which the invention may be performed.

Cells useful for the invention may be stored before use. Methods and protocols for preserving and storing of eukaryotic cells, and in particular mammalian cells, are well known in the art (cf., for example, Pollard, J. W. and Waker, J. M. (1997) Basic Cell Culture Protocols, Second Edition, Humana Press, Totowa, N.J.; Freshney, R. I. (2000) Culture of Animal Cells, Fourth Edition, Wiley-Liss, Hoboken, N.J.). Any method maintaining the biological activity of the isolated stem cells such as mesenchymal stem/progenitor cells, or progeny thereof, may be utilized in connection with the present invention. In one preferred embodiment, the cells are maintained and stored by using cryo-preservation.

### Cell-Sorting Techniques

Cells useful for the invention can be obtained using a variety of techniques. For example, a number of cell-sorting techniques by which cells are physically separated by reference to a property associated with the cell-antibody complex, or a label attached to the antibody can be used. This label may be a magnetic particle or a fluorescent molecule. The antibodies may be cross-linked such that they form aggregates of multiple cells, which are separable by their density. Alternatively the antibodies may be attached to a stationary matrix, to which the desired cells adhere.

In a preferred embodiment, an antibody (or other binding agent) that binds TNAP⁺, STRO-1⁺, VCAM-1⁺, THY-1⁺, STRO-2⁺, 3G5⁺, CD45⁺, CD146⁺ is used to isolate the cells. More preferably, an antibody (or other binding agent) that binds TNAP⁺ or STRO-1⁺ is used to isolate the cells.

Various methods of separating antibody-bound cells from unbound cells are known. For example, the antibody bound to the cell (or an anti-isotype antibody) can be labelled and then the cells separated by a mechanical cell sorter that detects the presence of the label. Fluorescence-activated cell sorters are well known in the art. In one embodiment, anti-TNAP antibodies and/or an STRO-1 antibodies are attached to a solid support. Various solid supports are known to those of skill in the art, including, but not limited to, agarose beads, polystyrene beads, hollow fiber membranes, polymers, and plastic petri dishes. Cells that are bound by the antibody can be removed from the cell suspension by simply physically separating the solid support from the cell suspension.

Super paramagnetic microparticles may be used for cell separations. For example, the microparticles may be coated with anti-TNAP antibodies and/or STRO-1 antibodies. The antibody-tagged, super paramagnetic microparticles may then be incubated with a solution containing the cells of interest. The microparticles bind to the surfaces of the desired stem cells, and these cells can then be collected in a magnetic field.

In another example, the cell sample is allowed to physically contact, for example, a solid phase-linked anti-TNAP monoclonal antibodies and/or anti-STRO-1 monoclonal antibodies. The solid-phase linking can comprise, for instance, adsorbing the antibodies to a plastic, nitrocellulose, or other surface. The antibodies can also be adsorbed on to the walls of the large pores (sufficiently large to permit flow-through of cells) of a hollow fiber membrane. Alternatively, the antibodies can be covalently linked to a surface or bead, such as Pharmacia Sepharose 6 MB macrobeads. The exact conditions and duration of incubation for the solid phase-linked antibodies with the stem cell containing suspension will depend upon several factors specific to the system employed. The selection of appropriate conditions, however, is well within the skill of the art.

The unbound cells are then eluted or washed away with physiologic buffer after allowing sufficient time for the stem cells to be bound. The unbound cells can be recovered and used for other purposes or discarded after appropriate testing has been done to ensure that the desired separation had been achieved. The bound cells are then separated from the solid phase by any appropriate method, depending mainly upon the nature of the solid phase and the antibody. For example, bound cells can be eluted from a plastic petri dish by vigorous agitation. Alternatively, bound cells can be eluted by enzymatically "nicking" or digesting an enzyme-sensitive "spacer" sequence between the solid phase and the antibody. Spacers bound to agarose beads are commercially available from, for example, Pharmacia.

The eluted, enriched fraction of cells may then be washed with a buffer by centrifugation and said enriched fraction may be cryopreserved in a viable state for later use according to conventional technology, culture expanded and/or introduced into the patient.

### Compositions and Administration Thereof

Typically, the cells are administered in a pharmaceutical composition comprising at least one pharmaceutically-acceptable carrier. The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. The phrase "pharmaceutically-acceptable carrier" as used herein means a pharmaceutically-acceptable material, composition or vehicle, such as a liquid or solid filler, diluent, excipient, or solvent encapsulating material.

Pharmaceutically acceptable carriers include saline, aqueous buffer solutions, solvents and/or dispersion media. The use of such carriers are well known in the art. The solution is preferably sterile and fluid to the extent that easy syringability exists. Preferably, the solution is stable under the conditions of manufacture and storage and preserved against the contaminating action of microorganisms such as bacteria and fungi through the use of, for example, parabens, chlorobutanol, phenol, ascorbic acid, thimerosal, and the like.

Some examples of materials and solutions which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other nontoxic compatible substances employed in pharmaceutical formulations.

The pharmaceutical compositions useful for the invention may comprise a polymeric carrier or extracellular matrix.

A variety of biological or synthetic solid matrix materials (i.e., solid support matrices, biological adhesives or dressings, and biological/medical scaffolds) are suitable for use in this invention. The matrix material is preferably medically acceptable for use in *in vivo* applications. Non-limiting examples of such medically acceptable and/or biologically or physiologically acceptable or compatible materials include, but are not limited to, solid matrix materials that are absorbable and/or non-absorbable, such as small intestine submucosa (SIS), e.g., porcine-derived (and other SIS sources); crosslinked or non-crosslinked alginate, hydrocolloid, foams, collagen gel, collagen sponge, polyglycolic acid (PGA) mesh, polyglactin (PGL) mesh, fleeces, foam dressing, bioadhesives (e.g., fibrin glue and fibrin gel) and dead de-epidermized skin equivalents in one or more layers.

Fibrin glues are a class of surgical sealants which have been used in various clinical settings. As the skilled address would be aware, numerous sealants are useful in compositions for use in the invention. However, a preferred embodiment of the invention relates to the use of fibrin glues with the cells described herein.

When used herein the term "fibrin glue" refers to the insoluble matrix formed by the cross-linking of fibrin polymers in the presence of calcium ions. The fibrin glue may be formed from fibrinogen, or a derivative or metabolite thereof, fibrin (soluble monomers or polymers) and/or complexes thereof derived from biological tissue or fluid which forms a fibrin matrix. Alternatively, the fibrin glue may be formed from fibrinogen, or a derivative or metabolite thereof, or fibrin, produced by recombinant DNA technology.

The fibrin glue may also be formed by the interaction of fibrinogen and a catalyst of fibrin glue formation (such as thrombin and/or Factor XIII). As will be appreciated by those skilled in the art, fibrinogen is proteolytically cleaved in the presence of a catalyst (such as thrombin) and converted to a fibrin monomer. The fibrin monomers may then form polymers which may cross-link to form a fibrin glue matrix. The cross-linking of fibrin polymers may be enhanced by the presence of a catalyst such as Factor XIII. The catalyst of fibrin glue formation may be derived from blood plasma, cryoprecipitate or other plasma fractions containing fibrinogen or thrombin. Alternatively, the catalyst may be produced by recombinant DNA technology.

The rate at which the clot forms is dependent upon the concentration of thrombin mixed with fibrinogen. Being an enzyme dependent reaction, the higher the temperature (up to 37°C) the faster the clot formation rate. The tensile strength of the clot is dependent upon the concentration of fibrinogen used.

Use of fibrin glue and methods for its preparation and use are described in U.S. Pat. No. 5,643,192. U.S. Pat. No. 5,643,192 discloses the extraction of fibrinogen and thrombin components from a single donor, and the combination of only these components for use as a fibrin glue. U.S. Pat. No. 5,651,982, describes another preparation and method of use for fibrin glue. U.S. Pat. No. 5,651,982, provides a fibrin glue with liposomes for use as a topical sealant in mammals.

Several publications describe the use of fibrin glue for the delivery of therapeutic agents. For example, U.S. Patent 4,983,393 discloses a composition for use as an intra-vaginal insert comprising agarose, agar, saline solution glycosaminoglycans, collagen, fibrin and an enzyme. Further, U.S. Patent 3,089,815 discloses an injectable pharmaceutical preparation composed of fibrinogen and thrombin and U.S. Patent 6,468,527 discloses a fibrin glue which facilitates the delivery of various biological and non-biological agents to specific sites within the body. Such procedures can be used in the methods of the invention.

Suitable polymeric carriers include porous meshes or sponges formed of synthetic or natural polymers, as well as polymer solutions. One form of matrix is a polymeric mesh or sponge; the other is a polymeric hydrogel. Natural polymers that can be used include proteins such as collagen, albumin, and fibrin; and polysaccharides such as alginate and polymers of hyaluronic acid. Synthetic polymers include both biodegradable and non-biodegradable polymers. Examples of biodegradable polymers include polymers of hydroxy acids such as polylactic acid (PLA), polyglycolic acid (PGA), and polylactic acid-glycolic acid (PLGA), polyorthoesters, polyanhydrides, polyphosphazenes, and combinations thereof. Non-biodegradable polymers include polyacrylates, polymethacrylates, ethylene vinyl acetate, and polyvinyl alcohols.

Polymers that can form ionic or covalently crosslinked hydrogels which are malleable are used to encapsulate cells. A hydrogel is a substance formed when an organic polymer (natural or synthetic) is cross-linked via covalent, ionic, or hydrogen bonds to create a three-dimensional open-lattice structure which entraps water molecules to form a gel. Examples of materials which can be used to form a hydrogel include polysaccharides such as alginate, polyphosphazines, and polyacrylates, which are crosslinked ionically, or block copolymers such as Pluronics™ or Tetronics™, polyethylene oxide-polypropylene glycol block copolymers which are crosslinked by temperature or pH, respectively. Other materials include proteins such as fibrin, polymers such as polyvinylpyrrolidone, hyaluronic acid and collagen.

In general, these polymers are at least partially soluble in aqueous solutions, such as water, buffered salt solutions, or aqueous alcohol solutions, that have charged side groups, or a monovalent ionic salt thereof. Examples of polymers with acidic side groups that can be reacted with cations are poly(phosphazenes), poly(acrylic acids), poly(methacrylic acids), copolymers of acrylic acid and methacrylic acid, poly(vinyl acetate), and sulfonated polymers, such as sulfonated polystyrene. Copolymers having acidic side groups formed by reaction of acrylic or methacrylic acid and vinyl ether monomers or polymers can also be used. Examples of acidic groups are carboxylic acid groups, sulfonic acid groups, halogenated (preferably fluorinated) alcohol groups, phenolic OH groups, and acidic OH groups. Examples of polymers with basic side groups that can be reacted with anions are poly(vinyl amines), poly(vinyl pyridine), poly(vinyl imidazole), and some imino substituted polyphosphazenes. The ammonium or quaternary salt of the polymers can also be formed from the backbone nitrogens or pendant imino groups. Examples of basic side groups are amino and imino groups.

Further, a composition used for the invention may comprise at least one therapeutic agent. For example, the composition may contain an analgesic to aid in treating inflammation or pain, another anti-angiogenic compound, or an anti-infective agent to prevent infection of the site treated with the composition. More specifically, non-limiting examples of useful therapeutic agents include the following therapeutic categories: analgesics, such as nonsteroidal anti-inflammatory drugs, opiate agonists and salicylates; anti-infective agents, such as antihelmintics, antianaerobics, antibiotics, aminoglycoside antibiotics, antifungal antibiotics, cephalosporin antibiotics, macrolide antibiotics, miscellaneous β-lactam antibiotics, penicillin antibiotics, quinolone antibiotics, sulfonamide antibiotics, tetracycline antibiotics, antimycobacterials, antituberculosis antimycobacterials, antiprotozoals, antimalarial antiprotozoals, antiviral agents, anti-retroviral agents, scabicides, anti-inflammatory agents, corticosteroid anti-inflammatory agents, antipruritics/local anesthetics, topical anti-infectives, antifungal topical anti-infectives, antiviral topical anti-infectives; electrolytic and renal agents, such as acidifying agents, alkalinizing agents, diuretics, carbonic anhydrase inhibitor diuretics, loop diuretics, osmotic diuretics, potassium-sparing diuretics, thiazide diuretics, electrolyte replacements, and uricosuric agents; enzymes, such as pancreatic enzymes and thrombolytic enzymes; gastrointestinal agents, such as antidiarrheals, gastrointestinal anti-inflammatory agents, gastrointestinal anti-inflammatory agents, antacid anti-ulcer agents, gastric acid-pump inhibitor anti-ulcer agents, gastric mucosal anti-ulcer agents, H2-blocker anti-ulcer agents, cholelitholytic agents, digestants, emetics, laxatives and stool softeners, and prokinetic agents; general anesthetics, such as inhalation anesthetics, halogenated inhalation anesthetics, intravenous anesthetics, barbiturate intravenous anesthetics, benzodiazepine intravenous anesthetics, and opiate agonist intravenous anesthetics; hormones and hormone modifiers, such as abortifacients, adrenal agents, corticosteroid adrenal agents, androgens, anti-androgens, immunobiologic agents, such as immunoglobulins, immunosuppressives, toxoids, and vaccines; local anesthetics, such as amide local anesthetics and ester local anesthetics; musculoskeletal agents, such as anti-gout anti-inflammatory agents, corticosteroid anti-inflammatory agents, gold compound anti-inflammatory agents, immunosuppressive anti-inflammatory agents, nonsteroidal anti-inflammatory drugs (NSAIDs), salicylate anti-inflammatory agents, minerals; and vitamins, such as vitamin A, vitamin B, vitamin C, vitamin D, vitamin E, and vitamin K.

Examples of other anti-angiogenic factors which may be used with the present invention, either in a single composition or as a combined therapy, include, but are not limited to, platelet factor 4; protamine sulphate; sulphated chitin derivatives (prepared from queen crab shells); Sulphated Polysaccharide Peptidoglycan Complex (SP-PG) (the function of this compound may be enhanced by the presence of steroids such as estrogen, and tamoxifen citrate); Staurosporine; modulators of matrix metabolism, including for example, proline analogs, cishydroxyproline, d,L-3,4-dehydroproline, Thiaproline, alpha,alpha-dipyridyl, aminopropionitrile fumarate; 4-propyl-5-(4-pyridinyl)-2(3H)-oxazolone; Methotrexate; Mitoxantrone; Heparin; Interferons; 2 Macroglobulin-serum; ChIMP-3; Chymostatin; Cyclodextrin Tetradecasulfate; Eponemycin; Camptothecin; Fumagillin; Gold Sodium Thiomalate; anticollagenase-serum; alpha2-antiplasmin; Bisantrene (National Cancer Institute); Lobenzarit disodium (N-(2)-carboxyphenyl-4-chloroanthronilic acid disodium); Thalidomide; Angostatic steroid; AGM-1470; carboxynaminolmidazole; and metalloproteinase inhibitors such as BB94.

In certain embodiments, the therapeutic agent may be a growth factor or other molecule that affects cell differentiation and/or proliferation. Growth factors that induce final differentiation states are well-known in the art, and may be selected from any such factor that has been shown to induce a final differentiation state. Growth factors for use in methods described herein may, in certain embodiments, be variants or fragments of a naturally-occurring growth factor.

Compositions useful for the present invention may include cell culture components, e.g., culture media including amino acids, metals, coenzyme factors, as well as small populations of other cells, e.g., some of which may arise by subsequent differentiation of the stem cells.

Compositions useful for the present invention may be prepared, for example, by sedimenting out the subject cells from the culture medium and resuspending them in the desired solution or material. The cells may be sedimented and/or changed out of the culture medium, for example, by centrifugation, filtration, ultrafiltration, etc.

The skilled artisan can readily determine the amount of cells and optional carrier(s) in compositions to be administered and according to the invention. In an embodiment, any additives (in addition to the active cell(s)) are present in an amount of 0.001 to 50% (weight) solution in phosphate buffered saline, and the active ingredient is present in the order of micrograms to milligrams, such as about 0.0001 to about 5 wt %, preferably about 0.0001 to about 1 wt %, still more preferably about 0.0001 to about 0.05 wt % or about 0.001 to about 20 wt %, preferably about 0.01 to about 10 wt %, and still more preferably about 0.05 to about 5 wt %. Of course, for any composition to be administered to an animal or human, and for any particular method of administration, it is preferred to determine therefore: toxicity, such as by determining the lethal dose (LD) and LD₅₀ in a suitable animal model e.g., rodent such as mouse; and, the dosage of the composition(s), concentration of components therein and timing of administering the composition(s), which elicit a suitable response. Such determinations do not require undue experimentation from the knowledge of the skilled artisan, this disclosure and the documents cited herein. And, the time for sequential administrations can be ascertained without undue experimentation.

The concentration of the cells in the composition may be at least about 5x10⁵ cells/mL, at least about 1x10⁶ cells/mL, at least about 5x10⁶ cells/mL, at least about 10⁷cells/mL, at least about 2x10⁷cells/mL, at least about 3x10⁷cells/mL, or at least about 5x10⁷cells/mL.

Compositions useful for the present invention can be for administration via, *inter alia*, localized injection, including catheter administration, systemic injection, localized injection, intravenous injection, intrauterine injection or parenteral administration. When administering a therapeutic composition described herein (e.g., a pharmaceutical composition), it will generally be formulated in a unit dosage injectable form (solution, suspension, emulsion).

### Production of Genetically Modified Cells

In one embodiment, the cells used in the invention are genetically modified. Preferably, the cells are genetically modified to produce a heterologous protein. Typically, the cells will be genetically modified such that the heterologous protein is secreted from the cells. However, in an embodiment the cells can be modified to express a functional non-protein encoding polynucleotide such as dsRNA (typically for RNA silencing), an antisense oligonucleotide or a catalytic nucleic acid (such as a ribozyme or DNAzyme).

Genetically modified cells may be cultured in the presence of at least one cytokine in an amount sufficient to support growth of the modified cells. The genetically modified cells thus obtained may be used immediately (e.g., in transplant), cultured and expanded *in vitro,* or stored for later uses. The modified cells may be stored by methods well known in the art, e.g., frozen in liquid nitrogen.

Genetic modification as used herein encompasses any genetic modification method which involves introduction of an exogenous or foreign polynucleotide into a cell described herein or modification of an endogenous gene within the cell. Genetic modification includes but is not limited to transduction (viral mediated transfer of host DNA from a host or donor to a recipient, either *in vitro or in vivo*), transfection (transformation of cells with isolated viral DNA genomes), liposome mediated transfer, electroporation, calcium phosphate transfection or coprecipitation and others. Methods of transduction include direct co-culture of cells with producer cells (Bregni et al., 1992) or culturing with viral supernatant alone with or without appropriate growth factors and polycations.

In a useful embodiment of the invention, the cells are genetically modified to contain a gene that disrupts or inhibits angiogenesis. The gene may encode a cytotoxic agent such as ricin. In another embodiment, the gene encodes a cell surface molecule that elicits an immune rejection response. For example, the cells can be genetically modified to produce α1, 3 galactosyl transferase. This enzyme synthesizes α1, 3 galactosyl epitopes that are the major xenoantigens, and its expression causes hyperacute immune rejection of the transgenic endothelial cells by preformed circulating antibodies and/or by T cell mediated immune rejection.

An exogenous polynucleotide is preferably introduced to the cell in a vector. The vector preferably includes the necessary elements for the transcription and translation of the inserted coding sequence. Methods used to construct such vectors are well known in the art. For example, techniques for constructing suitable expression vectors are described in detail in Sambrook et al., Molecular Cloning: A Laboratory Manual, Cold Spring Harbor Press, N.Y. (3rd Ed., 2000); and Ausubel et al., Current Protocols in Molecular Biology, John Wiley & Sons, Inc., New York (1999).

Vectors may include, but are not limited to, viral vectors, such as retroviruses, adenoviruses, adeno-associated viruses, and herpes simplex viruses; cosmids; plasmid vectors; synthetic vectors; and other recombination vehicles typically used in the art. Vectors containing both a promoter and a cloning site into which a polynucleotide can be operatively linked are well known in the art. Such vectors are capable of transcribing RNA *in vitro* or *in vivo,* and are commercially available from source such as Stratagene (La Jolla, Calif.) and Promega Biotech (Madison, Wis.). Specific examples include, pSG, pSV2CAT, pXtl from Stratagene; and pMSG, pSVL, pBPV and pSVK3 from Pharmacia.

Preferred vectors include retroviral vectors (see, Coffin et al., "Retroviruses", Chapter 9 pp; 437-473, Cold Springs Harbor Laboratory Press, 1997). Vectors useful in the invention can be produced recombinantly by procedures well known in the art. For example, WO94/29438, WO97/21824 and WO97/21825 describe the construction of retroviral packaging plasmids and packing cell lines. Exemplary vectors include the pCMV mammalian expression vectors, such as pCMV6b and pCMV6c (Chiron Corp.), pSFFV-Neo, and pBluescript-Sk+. Non-limiting examples of useful retroviral vectors are those derived from murine, avian or primate retroviruses. Common retroviral vectors include those based on the Moloney murine leukemia virus (MoMLV-vector). Other MoMLV derived vectors include, Lmily, LINGFER, MINGFR and MINT. Additional vectors include those based on Gibbon ape leukemia virus (GALV) and Moloney murine sarcoma virus (MOMSV) and spleen focus forming virus (SFFV). Vectors derived from the murine stem cell virus (MESV) include MESV-MiLy. Retroviral vectors also include vectors based on lentiviruses, and non-limiting examples include vectors based on human immunodeficiency virus (HIV-1 and HIV-2).

In producing retroviral vector constructs, the viral gag, pol and env sequences can be removed from the virus, creating room for insertion of foreign DNA sequences. Genes encoded by foreign DNA are usually expressed under the control a strong viral promoter in the long terminal repeat (LTR). Selection of appropriate control regulatory sequences is dependent on the host cell used and selection is within the skill of one in the art. Numerous promoters are known in addition to the promoter of the LTR. Non-limiting examples include the phage lambda PL promoter, the human cytomegalovirus (CMV) immediate early promoter; the U3 region promoter of the Moloney Murine Sarcoma Virus (MMSV), Rous Sacroma Virus (RSV), or Spleen Focus Forming Virus (SFFV); Granzyme A promoter; and the Granzyme B promoter. Additionally inducible or multiple control elements may be used. The selection of a suitable promoter will be apparent to those skilled in the art.

Such a construct can be packed into viral particles efficiently if the gag, pol and env functions are provided in trans by a packing cell line. Therefore, when the vector construct is introduced into the packaging cell, the gag-pol and env proteins produced by the cell, assemble with the vector RNA to produce infectious virons that are secreted into the culture medium. The virus thus produced can infect and integrate into the DNA of the target cell, but does not produce infectious viral particles since it is lacking essential packaging sequences. Most of the packing cell lines currently in use have been transfected with separate plasmids, each containing one of the necessary coding sequences, so that multiple recombination events are necessary before a replication competent virus can be produced. Alternatively the packaging cell line harbours a provirus. The provirus has been crippled so that although it may produce all the proteins required to assemble infectious viruses, its own RNA cannot be packaged into virus. RNA produced from the recombinant virus is packaged instead. Therefore, the virus stock released from the packaging cells contains only recombinant virus. Non-limiting examples of retroviral packaging lines include PA12, PA317, PE501, PG13, PSI.CRIP, RDI 14, GP7C-tTA-G10, ProPak-A (PPA-6), and PT67.

Other suitable vectors include adenoviral vectors (see, WO 95/27071) and adeno-associated viral vectors. These vectors are all well known in the art, e.g., as described in Stem Cell Biology and Gene Therapy, eds. Quesenberry et al., John Wiley & Sons, 1998; and U.S. Pat. Nos. 5,693,531 and 5,691,176. The use of adenovirus-derived vectors may be advantageous under certain situation because they are not capable of infecting non-dividing cells. Unlike retroviral DNA, the adenoviral DNA is not integrated into the genome of the target cell. Further, the capacity to carry foreign DNA is much larger in adenoviral vectors than retroviral vectors. The adeno-associated viral vectors are another useful delivery system. The DNA of this virus may be integrated into non-dividing cells, and a number of polynucleotides have been successful introduced into different cell types using adeno-associated viral vectors.

In some embodiments, the construct or vector will include two or more heterologous polynucleotide sequences. Preferably the additional nucleic acid sequence is a polynucleotide which encodes a selective marker, a structural gene, a therapeutic gene, or a cytokine/chemokine gene.

A selective marker may be included in the construct or vector for the purposes of monitoring successful genetic modification and for selection of cells into which DNA has been integrated. Non-limiting examples include drug resistance markers, such as G148 or hygromycin. Additionally negative selection may be used, for example wherein the marker is the HSV-tk gene. This gene will make the cells sensitive to agents such as acyclovir and gancyclovir. The NeoR (neomycin/G148 resistance) gene is commonly used but any convenient marker gene may be used whose gene sequences are not already present in the target cell can be used. Further non-limiting examples include low-affinity Nerve Growth Factor (NGFR), enhanced fluorescent green protein (EFGP), dihydrofolate reductase gene (DHFR) the bacterial hisD gene, murine CD24 (HSA), murine CD8a(lyt), bacterial genes which confer resistance to puromycin or phleomycin, and β-glactosidase.

The additional polynucleotide sequence(s) may be introduced into the cell on the same vector or may be introduced into the host cells on a second vector. In a preferred embodiment, a selective marker will be included on the same vector as the polynucleotide.

The present invention also encompasses genetically modifying the promoter region of an endogenous gene such that expression of the endogenous gene is up-regulated resulting in the increased production of the encoded protein compared to a wild type cell.

### Delivery of Anti-Cancer Agents

The term "anti-cancer agent" as used herein refers to any substance that inhibits or prevents the function of cancer cells and/or causes destruction of cancer cells. For example, an anti-cancer agent can be a cytotoxic agent. The anti-cancer agent may be conjugated to the stem cells, or progeny cells thereof. In an embodiment, the stem cells, or progeny cells thereof, comprise a transgene which encodes the anti-cancer agent.

The term "cytotoxic agent" as used herein refers to a substance that inhibits or prevents the function of cells and/or causes destruction of cells. The term is intended to include chemotherapeutic agents, and toxins such as enzymatically active toxins of bacterial, fungal, plant or animal origin, or fragments thereof. The cytotoxic agent can be conjugated to a cell targeting moiety such as an antibody. In a preferred embodiment, the cytotoxic agent is produced from a transgene of a cell that has been genetically modified.

In a preferred embodiment, the anti-cancer does not kill the stem cells, or progeny cells thereof. Examples of such agents include IL-2, interferon-y, anti-VEGF monoclonal antibodies and biologically active nucleic acids such as ribozymes and dsRNA which target important genes encoded by the cancer cells. Furthermore, the anti-cancer agent can be in a pro-toxic form that is processed (such as cleaved) *in vivo* to release the active anti-cancer agent.

The term "antibody" herein is used in the broadest sense and specifically covers monoclonal antibodies, polyclonal antibodies, multispecific antibodies (e.g., bispecific antibodies), and antibody fragments, so long as they exhibit the desired biological activity of, for example, binding a cancer cell. Antibodies may be murine, human, humanized, chimeric, or derived from other species.

"Antibody fragments" comprise a portion of a full length antibody, generally the antigen binding or variable region thereof. Examples of antibody fragments include Fab, Fab', F(ab')₂, and Fv fragments; diabodies; linear antibodies; fragments produced by a Fab expression library, anti-idiotypic (anti-Id) antibodies, CDR (complementary determining region), and epitope-binding fragments of any of the above which immunospecifically bind to cancer cell antigens, single-chain antibody molecules; and multispecific antibodies formed from antibody fragments.

Enzymatically active protein toxins and fragments thereof which can be used as cytotoxic agents include diphtheria A chain, nonbinding active fragments of diphtheria toxin, cholera toxin, botulinus toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, saporin, mitogellin, restrictocin, phenomycin, enomycin and the tricothecenes.

Examples of tumor-associated antigens or cell-surface receptors which can be targetted by an antibody-cytotoxic conjugate include, but are not limited to, BMPR1B (bone morphogenetic protein receptor-type 1B), E16, STEAP1 (six transmembrane epithelial antigen of prostate), 0772P (CA125, MUC16), MPF (MSLN, SMR, megakaryocyte potentiating factor, mesothelin), Napi3b (NAPI-3B, NPTIIb, SLC34A2, solute carrier family 34 (sodium phosphate), member 2, type II sodium-dependent phosphate transporter 3b), Sema 5b (FLJ10372, KIAA1445, Mm.42015, SEMA5B, SEMAG, Semaphorin 5b Hlog, sema domain, seven thrombospondin repeats (type I and type 1-like), transmembrane domain (TM) and short cytoplasmic domain, (semaphorin) 5B, PSCA hlg (2700050C12Rik, C530008016Rik, RIKEN cDNA 2700050C12, RIKEN cDNA 2700050C12 gene), ETBR (Endothelin type B receptor), MSG783 (RNF124, hypothetical protein FLJ20315), STEAP2 (IPCA-1, PCANAP1, STAMP 1, STEAP2, STMP, prostate cancer associated gene 1, prostate cancer associated protein 1, six transmembrane epithelial antigen of prostate 2, six transmembrane prostate protein), TrpM4 (BR22450, FLJ20041, TRPM4, TRPM4B, transient receptor potential cation channel, subfamily M, member 4), CRIPTO (CR, CR1, CRGF, CRIPTO, TDGF1, teratocarcinoma-derived growth factor), CD21 (CR2 (Complement receptor 2) or C3DR (C3d/Epstein Barr virus receptor) or Hs.73792), CD79b (CD79B, CD79p, 1Gb (immunoglobulin-associated beta), B29, FcRH2 (IFGP4, IRTA4, SPAP1A (SH2 domain containing phosphatase anchor protein 1a), SPAP1B, SPAP1C), HER2, NCA, MDP, IL20Rα, EphB2R, ASLG659, PSCA, GEDA, BAFF-R (B cell-activating factor receptor, BLyS receptor 3), CD22 (B-cell receptor CD22-β isoform), CD79α, CXCR5 (Burkitt's lymphoma receptor 1), HLA-DOB (Beta subunit of MHC class II molecule (Ia antigen) that binds peptides and presents them to CD4+ T lymphocytes), P2X5 (Purinergic receptor P2X ligand-gated ion channel 5), CD72 (B-cell differentiation antigen CD72, Lyb-2), LY64 (Lymphocyte antigen 64 (RP105)), FCRH1 (Fc receptor-like protein 1), IRTA2 (Immunoglobulin superfamily receptor translocation associated 2), and TENB2.

### EXAMPLES

The invention is hereinafter described by way of the following non-limiting Examples and with reference to the accompanying figures.

### Example 1: Induction of Choroidal Neovascularization (CNV)

### Materials and Methods

### Animal Preparation and Anaesthesia

A total of 15 nude rats were used during the study and the experimentation was performed in accordance with the Associate for Research in Vision and Ophthalmology (ARVO) Statement for the Use of Animals in Ophthalmic and Vision Research. All rats were housed in an isolated, sterile facility in cages (2 animals per cage) at a constant temperature of 22°C, with a 12:12 hour light/dark cycle (light on at 0800 hours) and food and water were available *ad libitum.* Rats were anaesthetised by intramuscular injection of xylazine (6 mg/kg, Bayer AG, Germany) and ketamine (50 mg/kg, Lambert Company, USA) injection. The pupils were dilated with 2.5% phenylephrine (Chauvin Pharmaceuticals Ltd, Romford, Essex) and 1% Mydriacyl (Alcon, Belgium), at least 10 minutes before laser photocoagulation, intravitreal injections and photography. Aseptic techniques were used throughout the course of the experiments.

### Krypton Laser Irradiation

Krypton laser irradiation (647.1nm, coherent Radiation System, CA, USA) was delivered to the left eye of each animal through a Zeiss slit lamp with a hand-held coverslip serving as a contact lens. A total of 6-11 laser bums were applied to each eye surrounding the optic nerve at the posterior pole at a setting of 100 µm diameter, 0.1 seconds duration and 150 mW intensity.

### Color Fundus Photography (CFP)

Animals were anaesthetised prior to photography as mentioned in animal preparation and anaesthesia. The pupils were dilated with 2.5% phenylephrine (Chauvin Pharmaceuticals Ltd, Romford, Essex) and 1% Mydriacyl (Alcon, Belgium), at least 10 minutes before photography. The rat fundus photography was performed using a small animal fundus camera (Kowa Genesis Tokyo, Japan) using Kodak Elite 200 ASA slide film.

### Fluoroscein Angiography (FA)

Fluorescein angiography was performed on all rats through intraperitoneal injection of 0.1 ml 10% sodium fluorescein. The retinal vasculature was photographed using the same camera as CFP (Kowa Genesis Tokyo, Japan) but with a barrier filter for fluorescein angiography added. Single photographs were taken at 0.5-1 minute intervals using Kodak Tmax 400 ASA monochrome professional film immediately after the administration of sodium fluorescein.

### Formation and Extent of Choroidal Neovascularization (CNV)

The formation and extent of CNV in the eye using this model was monitored using CFP and FA. Anaesthetized animals were injected intraperitoneally with 0.3 to 0.4 ml of 10% sodium fluorescein and the eyes photographed using fluorescence fundus photography. The extent of fluorescein leakage was calculated using the densitometry method (Marano and Rakoczy, 2005). Briefly, the fluorescein angiograms were scanned into a computer using a LS-4000 ED film scanner (Nikon Corp., Tokyo, Japan) for import into Quantity One® basic densitometry software (Bio-Rad, CA, USA). The relative fluorescence (rf) for each lesion was calculated and these values were subsequently converted into severity scores by comparison to a severity template, which was used to generate rf intervals whereby a rf of 0 - 20, no leakage; 21 - 70, mild leakage; 71 - 120, moderate leakage and > 121, severe leakage. The mean severity scores from each of the time points were compared by ANOVA with a post hoc Fishers LSD analysis. Differences were considered significant at p < 0.05. In addition, the frequency of each lesion score was counted, tabulated and represented graphically.

### Histological Studies

Five rats were euthanased at 7 days post injection with an overdose of sodium pentobarbital (Nembutal) and the remaining 10 euthanased at the completion of the experiment on day 28. Lasered eyes from two of the animals sacrificed at day 7 were embedded in OCT medium and frozen on liquid N₂ for fluorescence microscopy and immunohistochemistry using the CD68 antibody for macrophage detection. Sections were examined under UV light and images captured using an Olympus microscope (Olympus, Tokyo, Japan) and an Olympus DP70 video camera at 20x magnification with a 2 second exposure time. The eyes from the remaining animals euthanized at day 7 and 28 were enucleated and fixed for 4 hours in 10% neutral buffered saline or 4% paraformaldehyde. After routine processing through graded alcohol, the eyes were embedded in paraffin and sectioned at 5 µm, mounted on silanated slides and stained with haematoxylin and eosin (H&E) for histopathological examination.

### Results

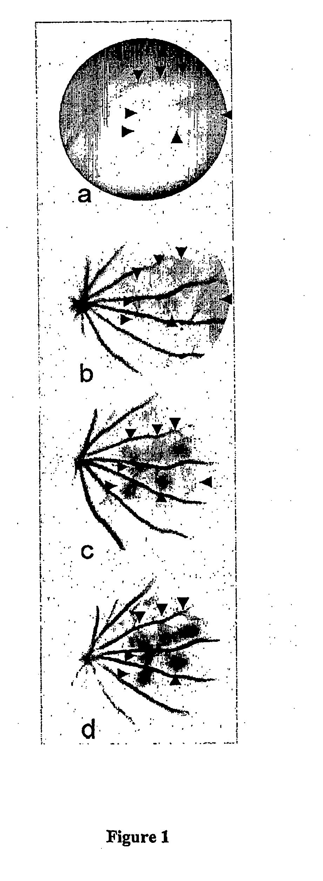

Colour fundus photography was used to confirm the presence of laser lesions (Figure 1 a). Fluorescein angiography was subsequently performed to document the level of fluorescein leakage from each of the lesions, which provides an indication of the level of CNV development. Initial clinical observations at day 7 post laser photocoagulation indicated a zero to mild level of leakage for most of the lesions (Figure 1b). Following this time point, at day 14 a dramatic increase in the level of leakage was observed (Figure 1c) and by day 28 this level of leakage seemed to increase once again but only slightly compared to the day 7 - 14 increase (Figure 1d).

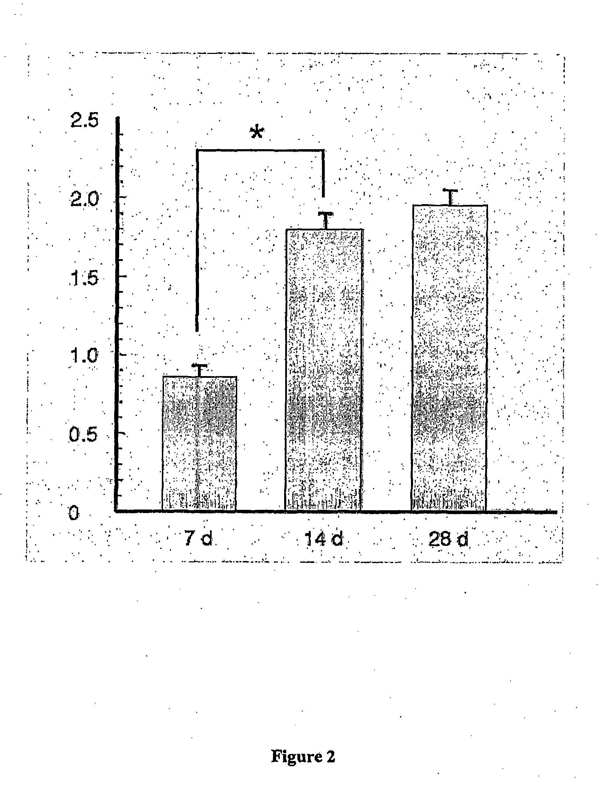

Severity scores were subsequently calculated for each of the lesions at 7, 14 and 28 days post laser photocoagulation to provide a mean severity score for each time point (Figure 2). At 7 days, the mean leakage score was well within the zero to mild scale (0.86 ± 0.07) indicating that leakage was minimal at this point. This finding shows that fluorescein leakage due to CNV does not occur in the first 3 to 4 days following laser photocoagulation in other rodent species. At day 14, the mean leakage score significantly increased from the 7 day level (1.8 ± 0.1 p < 0.01) as CNV development progressed. At day 28 post laser photocoagulation, the mean severity score of the lesions had again increased, but not significantly compared to the 14 day score (1.96 ± 0.1; p > 0.01) showing that leakage due to CNV is peaking around this time point.

A second analysis was performed, which examined the frequency of each lesion score for each time point (Table 1 and Figure 3). At day 7, over 87% of the lesions were exhibiting zero to mild leakage (29.3% zero and 58.6% mild) with the remaining showing moderate to severe leakage. By day 14, there was a dramatic shift in the frequency of each of the lesion scores with the zero to mild scores being reduced 32.4% of the total lesions (7% and 25.4% respectively). The majority (47.9%) of the lesions were in the moderate range and the number of lesions exhibiting strong leakage increased from 1% at day 7 to 19.7% to give a total of 67.6% of lesions in the moderate to severe score. By day 28, the number of lesions progressing to higher scores had slowed. The number of zero ranked lesions had dropped to 2.8%, which is typical of this model and expected, as a small percentage of laser lesions fail to rupture Bruch's membrane and therefore never develop CNV. The majority of the scores (70%) remain in the moderate to strong rank (42.2% and 28.2% respectively), which is again typical of this model.

**Table 1. Frequency values of each lesion score at each time point**

| | **Severity score (% of total lesions scored)** | | | |
|---|---|---|---|---|
| **Group** | **0** | **1** | **2** | **3** |
| 7d (n = 99) | 29,3% (29) | 58.6% (58) | 11.1% (11) | 1% (1) |
| 14d (n = 71) | 7% (5) | 25.4% (18) | 47.9% (34) | 19.7% (14) |
| 28d (n = 71) | 2.8% (2) | 26.8% (19) | 42.2% (30) | 28.2% (20) |

| | | | | |
|---|---|---|---|---|
| 0 = no leakage; 1 = mild leakage; 2 = moderate leakage; 3 = severe leakage. | | | | |

Histological sections of the eyes shows the appearance of a normal, unlasered retina (Figure 4a) plus the appearance of the retina following laser photocoagulation, showing the lesions and breakage of Bruch's membrane (Figure 4b, indicated a bar), which is essential for the effectiveness of this model. During the examination of the sections, the presence of red blood cells were identified within the lesion site (Figure 4c, arrowheads), which was an indication of early CNV development. By day 28, the presence of red blood cells appeared to have increased by a noticeable proportion (Figure 4d and e, arrowheads). These observations are in correlation with the calculated severity scores and frequency distributions of each leakage score.

Detection of the CD68 antigen (macrophages) was performed on the frozen sections using a FITC tagged secondary antibody for visualisation under a UV light microscope. Visualisation of non-detected sections (Figure 5) shows the level of autofluorescence exhibited by the retinal tissue, where the remnants of the RPE layer (shows as yellow) can be seen throughout the lesion site (arrows). Following depigmentation and immuno-detection, no visible signs of macrophages could be detected (data not shown).

### Discussion

It was determined that the induction of choroidal neovascularization by krypton laser photocoagulation as described herein would be a suitable model to test for an agents ability to treat ocular neovascularization.

### Example 2: Effect of Human Cells on The Development of CNV

### Materials and Methods

Animals were prepared and anaesthetised, and krypton laser irradiation was performed, as in Example 1. The evaluation of CNV was also performed as per Example 1.

### Preparation of Cells

Bone marrow (BM) is harvested from healthy normal adult volunteers (20-35 years old), in accordance with procedures approved by the Institutional Ethics Committee of the Royal Adelaide Hospital. Briefly, 40 ml of BM is aspirated from the posterior iliac crest into lithium-heparin anticoagulant-containing tubes. BMMNC are prepared by density gradient separation using Lymphoprep™ (Nycomed Pharma, Oslo, Norway) as previously described (Zannettino et al., 1998). Following centrifugation at 400 x g for 30 minutes at 4°C, the buffy layer is removed with a transfer pipette and washed three times in "HHF", composed of Hank's balanced salt solution (HBSS; Life Technologies, Gaithersburg, MD), containing 5% fetal calf serum (FCS, CSL Limited, Victoria, Australia).

TNAP+ cells were subsequently isolated by magnetic activated cell sorting as previously described (Gronthos et al., 2003; Gronthos et al., 1995). Briefly, approximately 1-3 x 10⁸ BMMNC are incubated in blocking buffer, consisting of 10% (v/v) normal rabbit serum in HHF for 20 minutes on ice. The cells are incubated with 200µl of a 10µg/ml solution of STRO-3 mAb in blocking buffer for 1 hour on ice. The cells are subsequently washed twice in HHF by centrifugation at 400 x g. A 1/50 dilution of goat anti-mouse γ-biotin (Southern Biotechnology Associates, Birmingham, UK) in HHF buffer is added and the cells incubated for 1 hour on ice. Cells are washed twice in MACS buffer (Ca²⁺ - and Mn²⁺ -free PBS supplemented with 1% BSA, 5 mM EDTA and 0.01% sodium azide) as above and resuspended in a final volume of 0.9 ml MACS buffer.

One hundred µl streptavidin microbeads (Miltenyi Biotec; Bergisch Gladbach, Germany) are added to the cell suspension and incubated on ice for 15 minutes. The cell suspension is washed twice and resuspended in 0.5 ml of MACS buffer and subsequently loaded onto a mini MACS column (MS Columns, Miltenyi Biotec), and washed three times with 0.5 ml MACS buffer to retrieve the cells which did not bind the STRO-3 mAb (deposited on 19 December 2005 with American Type Culture Collection (ATCC) under accession number PTA-7282 - see co-pending International application WO 2006/108229). After addition of a further 1 ml MACS buffer, the column is removed from the magnet and the TNAP-positive cells are isolated by positive pressure. An aliquot of cells from each fraction can be stained with streptavidin-FITC and the purity assessed by flow cytometry.

Primary cultures are established from the MACS isolated TNAP+ cells by plating in α-MEM supplemented with 20% fetal calf serum, 2mM L-glutamine and 100µm L-ascorbate-2-phosphate as previously described (Gronthos et al., 1995).

Cells prepared as described herein are also referred to as Human Mesenchymal Precursor Cells (HMPCs).

### Intravitreal Injection

Intravitreal injections were performed by inserting a 30-gauge needle into the vitreous at a site 1 mm posterior to the limbus of the eye. Insertion and infusion were performed and directly viewed through an operating microscope. Care was taken not to injure the lens or the retina. Rats were injected in the left eye with 2 µl of the cells (5.625 x 10⁴ cells / µl) on the day following laser photocoagulation, and attempts were made to place them in the superior and peripheral vitreous cavity.

### Histological Studies

Rats were euthanased with an overdose of sodium pentabarbital (Nembutal) at the completion of the experiment on day 28. All eyes from these animals were enucleated and fixed for 4 hours in 10% neutral buffered saline or 4% paraformaldehyde. After routine processing through graded alcohol, the eyes were embedded in paraffin and sectioned at 5 µm, mounted on silanated slides and stained with haematoxylin and eosin (H&E) for histopathological examination.

### Results

CFP was used to confirm the presence of laser lesions in the treated eyes of the animals and FA was subsequently performed immediately following CFP to document the level of fluorescein leakage from each of the lesions (Figure 6). In a control eye that was injected with cells but not laser photocoagulated, the fundus (Figure 6a) and the vasculature (Figure 6b) appear to be normal. In the treated eyes, the laser lesions were clearly visible 7 days post injection (Figure 6c, arrows) and FA indicated a predominance of zero to mild levels of leakage for most of the lesions (Figure 6d, arrows). Following this time point, at day 14 the lesions were still clearly visible using CFP (Figure 6e, arrows). However, initial observations using FA (Figure 6f) suggested that leakage had not noticeably increased as was observed in the 14 day control eyes of Example 1. This was also found to be the case at day 28 post injection, whereby the lesions remained clearly visible (Figure 6g arrows) while the leakage did not seem to have increased by a noticeable amount (Figure 6h).

Severity scores were subsequently calculated for each of the lesions at 7, 14 and 28 days post injection of the cells to provide a mean severity score for each time point (Figure 7). At 7 days, the mean leakage score was well within the zero to mild scale (0.88 ± 0.14) indicating that leakage was minimal at this point. This result was not significantly different from the control animals (p > 0.05) measured at the same time point from Example 1 (0.86 ± 0.07). At day 14, the mean leakage score did not increase significantly from the 7 day level (0.93 ± 0.16, p > 0.05) and was significantly lower than the lesion score of the 14 day control group (1.8 ± 0.1, p < 0.01). Similarly, at day 28 post injection, the mean severity score of the lesions had increased, but not significantly compared to the 7 day score (1.18 ± 0.17; p > 0.01) and was significantly lower than the comparative score of the control group (1.96 ± 0.1, p<0.01).

Similar to Example 1, the frequency of each lesion score for each time point was examined (Table 2 and Figure 8). At day 7, over 73% of the lesions were exhibiting zero to mild leakage with the remaining showing moderate leakage. By day 14, the frequency distributions did not alter appreciably with 39.9% of the lesions still showing zero leakage. There was a slight decrease in lesions with mild leakage (35.3% to 28.6%) and a small increase in lesions with moderate leakage (26.5% to 32.1%), no lesions presented with severe leakage. By day 28, a small number of lesions had progressed to the severe rating (8.7%), with a large percentage still exhibiting zero leakage (32.4%). These data are in contrast to the control group where at 14 and 28 days, the percentage of zero leakage scores had fallen to 7% and 2.8% respectively and the number of severe lesions were 19.7% to 28.2% respectively.

**Table 2. Frequency values of each lesion score at each time point**

| | **Severity score (% of total lesions scored)** | | | |
|---|---|---|---|---|
| **Group** | **0** | **1** | **2** | **3** |
| 7d (n = 34) | 38.2% (13) | 35.3% (12) | 26.5% (9) | 0% (0) |
| 14d (n = 28) | 39.3% (11) | 28.6% (8) | 32.1% (9) | 0% (0) |
| 28d (n = 34) | 32.4% (11) | 26.5% (9) | 32.4% (11) | 8.7% (3) |

| | | | | |
|---|---|---|---|---|
| 0 = no leakage; 1 = mild leakage; 2 = moderate leakage; 3 = severe leakage. | | | | |

Histological sections of eyes presented in Figure 9 show the presence of large numbers of macrophages (Figure 9a and b, arrow), in addition to an absence of any discernable retinal tissue. Sections of eyes possessed few macrophages (Figure 9b), no sign of infection could be detected and the retinal tissue appeared otherwise normal. In addition, no sign of developing blood vessels could be detected within the lesion site.

### Discussion

The retinas of the left eye of 15 nude rats were laser photocoagulated with 6 - 10 lesions. On the following day, the lasered eyes were injected with 2 µl of cells (5.625 x 10⁴ cells / µl) and subjected to ophthalmic evaluation, CFP and FA at 3 time points (7, 4 and 28 days post injection).

Initial clinical observations indicated that leakage due to CNV development within the lesions did not appear to increase with time. Subsequent densitometry on the lesions using the FA's revealed this to be the case as no significant increase in mean fluorescein leakage was calculated from 7 to 28 days. In addition, no blood vessels could be detected within the burn sites when viewing the histological sections, indicating inhibition of angiogenesis.

### Example 3: Optimising stem cell therapy in a laser-induced rodent model of CNV Material and Methods

### Animals

Twenty 8-10 week old female CBH-rnu/Arc rats were obtained from the Animal Resource Centre, Western Australia for this study. Homozygous CBH-rnu/ARc rats have little or no hair and are athymic and have similar dysgenesis to nude mutation in mice. Their cell-mediated immunity is greatly reduced or absent with a marked reduction of T-lymphocyte function and they are extremely susceptible to inflection with *Clostridium piliformi.*

The CBH-rnu/Arc rats were housed in filter-topped cages on a bedding of chaff at a constant temperature of 22°C and with a 12:12 hour light/dark cycle (light on at 0800 hours). Food and water were available *ad libitum.* The physical state of the rats (eg. weight, movement etc) was monitored throughout the study.

### Procedures

All procedures where performed in accordance to the guidelines of the Animal Ethics and Experimentation Committee of The University of Western Australia and the Association for Research in Vision and Ophthalmology Statement.

### Anaesthesia and Pupil Dilation

All clinical photography, intravitreal injection and laser photocoagulation were performed with the rats under general anaesthesia which involved intramuscular injection of a mixture of ketamine (50 mg/kg, Lambert Company, USA) and xylaxine (6 mg/kg, Bayer AG, Germany). The pupil was dilated with 2.5% phenylephrine (Chauvin Pharmaceutical Ltd., Romford, Essex) and 1% Mydriacyl (Alcon, Belgium) eye drops.

### Preparation of Cells

HMPCs were prepared as described above in Example 2.

### Laserphotocoagulation

A krypton laser (647.1nm, Coherent Radiation System, CA, USA) was applied to the fundus through a Zeiss slit lamp with a hand held coverslip serving as a contact lens to the left eye of each CBH-rnu/Arc rat. Eight to thirteen laser photocoagulations were performed in each eye between the major retinal vessels around the optic nerve using a setting of 100 µm diameter, 0.1 second exposure time, and 150 mW power. Formation of a bubble was used as an indication for successful rupturing of Bruch's membrane.

### Clinical Photography

Colour fundus photography was performed using a modified portable small animal fundus camera (Genesis; Kowa, Tokyo, Japan) with a condensing lens (Volk Superfield, Volk Optical, Mentor, OH) interposed to increase the field of view. Fluorescein angiography was performed following intraperitoneal injection of the rats with 100 µl of a 10% sodium fluorescein solution using confocal scanning laser ophthalmoscopy (Heidelberg Retinal Angiograph; Heidelberg Engineering, Carlsbad, CA).

### Intravitreal Injection

The conjunctiva was cut and a 30-gauge needle was used to make an initial puncture of the exposed sclera. A 32-gauge needle attached to a 5 µl Hamilton syringe was then passed through the puncture in the sclera into the vitreous cavity. The advancement of the needle, controlled using a micro-delivery system, was directly observed under an operating microscope and the agent was delivered when the needle tip reached the vitreous cavity. The needle was kept in the vitreous cavity for about 1 minute before being withdrawn and antibiotic ointment (0.5% chloramphenicol drop, Chauvin Pharmaceutical Ltd.) was applied to prevent infection. The injected eyes were observed daily and chloramphenicol drops were applied for the first three days following injection.

### Histological Assessment

Organs were collected following sacrifice of the rats at day 30 post-injection and fixed in either Bouin's Fixative (eye and optic nerve) or in buffered 3.7% formaldehyde. The organs were washed 3 times in phosphate-buffered saline (PBS, pH 7.2-7.4), dehydrated in graded solutions of ethanol and embedded in paraffin wax. Selected tissues were sectioned and stained with haematoxylin and eosin for histological examination (H&E). For morphometric measurement of choroidal neovascular membrane thickness, ten laser lesions were randomly selected from the Profreeze^{™}-(Lonza, Basel, Switerland, which comprises CDM NAO Freezing Medium, 7.5% DMSO and 50% Alpha MEM) and cell-injected eyes. The maximum vertical meridian passing through this spindle-shaped scar was measured as the thickness.

### Experimental Design

Twenty CBH-rnu/Arc rats were examined physically and anaesthetised. Following dilation of the pupil, laser photocoagulation was performed in the left eye of each rat. The formation of a bubble following laser photocoagulation was used as an indication of rupture of Bruch's membrane. A total of between 8 and 13 laser photocoagulations were performed in each eye and distribution of the laser photocoagulations was immediately imaged using colour fundus photography (day -1).

A day following laser photocoagulation (day 0), 10 laser-photocoagulated eyes were intravitreally injected with 3 µl Profreeze and the remaining 10 laser-photocoagulated CBH-rnu/Arc rat eyes were intravitreally injected with 3 µl HMPCs. Antibiotic ointment was applied to the injected eye. The rats were examined daily for the next 3 days and antibiotic ointment was applied everyday for the first 3 days post-injection.

At day 7, day 14 and day 28 post-injection, clinical photography (colour fundus photography and fluorescein angiography) was performed on the treated eyes. The CBH-rnu/Arc rats were sacrificed at day 30 post-injection by carbon dioxide asphyxiation and the eyes and organs were harvested. All eyes were fixed in Bouin's Fixative while the organs were immersed in buffered 3.7% formaldehyde. The tissue samples were then dehydrated in graded solutions of ethanol and embedded in paraffin wax. Selected tissues were sectioned and stained with H&E.

### Results and Discussion

### Physical Status of CBH-rnu/Arc rats Used in the Study

The CBH-rnu/Arc rats were healthy and were within a healthy weight range before and during the course of the study. Although they were subjected to different procedures during the study, they fed well and moved normally upon complete recovery from anaesthesia. Some had slight bruising at the site of intramuscular injection but this did not affect their movement, feeding and behaviour.

The eyes of the 20 rats used in the study were normal at the start of the study. Left and right eyes of each rat were of equal size. Their pupils dilated easily and their conjunctiva, cornea, iris and lens were all normal. All had clear vitreous, normal fundus and retinal vasculature with no haemorrhage at the start of the study.

Immediately after laser photocoagulation, the vitreous was clear and the laser photocoagulated spots were clear and well defined. In a few eyes, haemorrhage due to accidental placement of laser photocoagulation onto a retinal vessel was occasionally observed.

### Analysis of Toxic effect of Profreeze and Cells in eyes of CBH-rnu/Arc rats

Profreeze and cells were injected into the vitreous of the photocoagulated eye of each rat. All eyes were normal immediately following injection.

Colour fundus photography and slit lamp photography of the eyes at day 7, 14 and 28 post-injection showed that, with the exception of rats #10, #24 and #25, the presence of Profreeze and cells did not have any adverse effect on the eye: the cornea was normal, the anterior chamber and vitreous were both clear. Rat #10 and rat #24 had vitreous that was cloudy at day 7 post injection and this inhibited imaging at this time point. However, the vitreous appeared clear again at days 14 and 28. Rat #25 had anterior uveitis at day 7 and a partial cataract was observed at the later time points. The partial cataract was most likely caused by accidental damage to the lens during the injection. Clinical imaging of some eyes could not be carried out due to anaesthesia-related cataract.

In order to evaluate if Profreeze and cells had any local or systemic toxic effect following intravitreal delivery, tissues and organs were harvested at 30 days post-injection. Based on the necropsy report, pathological changes were detected in the tissues and organs at approximately the same frequency in both the Profreeze- and cell-injected rats, suggesting that the presence of the injected cells did not, by itself, have any adverse effect.

### Analysis of efficacy of Profreeze and Cells

The newly formed membranes in the laser-induced rat CNV model were quantified by evaluating fluorescein leakage using fluorescein angiography. The percentage of laser photocoagulation with fluorescein leakage at each time point was evaluated for the Profreeze-injected and cell-injected groups.

For calculating the success rate of developing CNV in the laser-induced CNV model, only eyes with fluorescein angiograms that could be used for the assessment of fluorescein leakage at day 7 post-laser photocoagulation were used. For this reason rats # 24, #16, #19 and #25 were excluded from this assessment. From a total of 167 laser photocoagulations performed, 122 or 73.1% had fluorescein leakage at day 7 (Table 3). This percentage fell within the range reported previously by other groups (Yanagi et al., 2002; El Bradey et al., 2004; Zou et al., 2006)).

**Table 3: Summary of laser photocoagulations delivered and number of laser photocoagulations with fluorescein leakage at the different time points post-injection. # NA: not available due to anaesthesia-related cataract. NA: not available.**

| **Animal ID** | **Treatment** | **Number of laser spots delivered** | **Number of laser spots with fluorescein leakage at the following times post injection** | | |
|---|---|---|---|---|---|
| | | | **7 days** | **14 days** | **28 days** |
| 1 | HMPC | 8 | 7 | 7 | 1 |
| 2 | HMPC | 11 | 10 | 8 | 3 |
| 3 | HMPC | 12 | 8 | 9 | 6 |
| 4 | HMPC | 11 | 9 | 10 | 5 |
| 5 | HMPC | 12 | 10 | 5 | 3 |
| 6 | HMPC | 13 | 9 | 5 | 0 |
| 7 | HMPC | 9 | 5 | 6 | 3 |
| 8 | HMPC | 11 | 6 | #NA | 2 |
| 10 | HMPC | 10 | 6 | NA | NA |
| 24 | HMPC | 9 | NA | 8 | 8 |
| | | | | | |
| 12 | Profreeze | 9 | 8 | 8 | 8 |
| 13 | Profreeze | 11 | 9 | 9 | 9 |
| 14 | Profreeze | 12 | 10 | 9 | 8 |
| 16 | Profreeze | 10 | NA | 7 | 8 |
| 17 | Profreeze | 11 | 6 | 10 | 10 |
| 18 | Profreeze | 8 | 6 | 6 | 3 |
| 19 | Profreeze | 12 | NA | 9 | 6 |
| 20 | Profreeze | 10 | 7 | 6 | #NA |
| 23 | Profreeze | 9 | 6 | 6 | 7 |
| 25 | Profreeze | 9 | NA | NA | 1 |

In calculating the change in percentage of photocoagulations with fluorescein leakage with time, only eyes with the complete set of fluorescein angiograms were included. In the Profreeze-injected group, rat #16, rat #19, rat #20 and rat #23 were excluded and in the cell-injected group, rat #8, rat #10 and rat #24 were excluded from this assessment.

In the Profreeze-injected group, 75% of laser photocoagulations had fluorescein leakage at day 7 post-laser photocoagulation and this increased to 80% at day 14 and dropping back to 75% at 28 days post-laser photocoagulation (Figure 10). In contrast, in the HMPC-injected eyes, 76.3% of the photocoagulations had fluorescein leakage and this dropped to 65.8% at 14 days and further to 30.3% at day 28 post-laser photocoagulation (Figure 10). This suggests that the presence of the cells has an effect on inhibiting neovascularisation in this model.

From light microscopic analysis of H&E-stained sections of the eyes, foci of damage (laser lesions) were present in all Profreeze- and HMPC-injected eyes. Such lesions consisted of thinning of outer plexiform and outer nuclear layers. Pigmented cells and small blood vessels were found in the outer nuclear layer. Focal thickening of the retinal pigment epithelial layer was also seen, together with some focal increase in concentration of small blood vessels. Presence of neovascular membranes, which present as spindle-shaped subretinal fibrovascular scars in the laser lesions with single or multilayered retinal pigment epithelial cells, was obvious and could be distinguished from adjacent normal structure in the photocoagulated eyes (Figure 11).

A comparison of the maximum thickness of 10 randomly selected choroidal neovascular membranes from the eyes injected with Profreeze (n=6) and cells (n=7) was carried out. The average thickness of the choroidal neovascular membranes was 35.1 ± 10.2 µm in the Profreeze-injected group and 16.3 ± 4.7 µm in the cell-injected group (Figure 12). The difference in average thickness was statistically significant (*p*<0.001, Student's T-test).

As the thickness of choroidal neovascular membrane in the laser-induced CNV model has been used as one of the parameters to assess efficacy of anti-angiogenic activity of compounds or molecules (Lai et al., 2002; Berglin et al., 2003; Krzystolik et al., 2002; Ciulla et al., 2003), the reduction in thickness of choroidal neovascular membrane in the HMPC-injected eyes indicates that HMPCs has an effect of suppressing laser-induced choroidal neovascularisation.

It will be appreciated by persons skilled in the art that numerous variations and/or modifications may be made to the invention as shown in the specific embodiments without departing from the invention as defined in the independent claims. The present embodiments are, therefore, to be considered in all respects as illustrative and not restrictive.

### REFERENCES

Angiolillo, A. L. et al. (1995) JExp Med. 182: 155-162.
Berglin, L. et al. (2003) Invest Ophthalmol Vis Sci 44: 403-408.
Bregni, M. et al. (1992) Blood 80:1418-1422.
Cao, Y. et al. (1995) J Exp Med. 182: 2069-2077.
Chen, C. et al. (1995) Cancer Res. 55: 4230-4233.
Ciulla, T.A. et al. (2003) Br J Ophthalmol 87:1032-1037.
Clapp, C. et al. (1993) Endocrinology. 133: 1292-1299.
El Bradey, M et al (2004) J Ocul Pharmacol Ther 20: 217-236.
Folkman, J. (1971) NEngl J Med. 285: 1182-1186.
Good, D. J. et al. (1990) Proc Natl Acad Sci USA. 87: 6624-6628.
Gronthos, S. and Simmons, P.J. (1995) Blood 85: 929-940.
Gronthos, S. et al. (2003) J Cell Sci 116: 1827-1835.
Gupta, S. K et al. (1995) Proc Natl Acad Sci USA. 92: 7799-7803.
Kandel, J. et al. (1991) Cell. 66: 1095-1104.
Krzystolik, M.G. et al. (2002) Arch Ophthalmol 120: 338-346.
Lai, Y.K. et al. (2002) Gene Ther 9: 804-813:
Maione, T. E. and Sharpe, R. J. (1990) Trends Pharmacol Sci. 11: 457-461.
Marano, R.J. and Rakoczy P.E. (2005) Clin Experiment Ophthalmol 33: 81-89.
O'Reilly, M. S. et al. (1994) Cell. 79: 315-328.
O'Reilly, M. S. et al. (1997) Cell. 88: 277-285.
Strieter, R. M. et al. (1995) Biophys Biochem Res Commun. 210: 51-57.
Voest, E. E. et al. (1995) J Natl Cancer Inst. 87: 581-586.
Yanagi, Y. et al. (2002) Invest Ophthalmol Vis Sci 43: 3495-3499..
Zannettino, A.C. et al. (1998) Blood 92: 2613-2628.
Zou, Y. et al. (2006) J Ocul Pharmacol Ther 22: 19-25.

## Claims

1. Use of adult STRO-1^{bright} mesenchymal precursor cells (MPC), or expanded cells thereof, in the manufacture of a medicament for treating or preventing macular degeneration or diabetic retinopathy in a subject.

2. Use according to claim 1, wherein the macular degeneration is dry age-related macular degeneration or wet age-related macular degeneration.

3. Use according to claim 1 or claim 2, wherein the stem cells are obtained from bone marrow.

4. Use according to any one of claims 1 to 3, wherein at least some of the cells are genetically modified.

5. Adult STRO-1^{bright} Mesenchymal precursor cells (MPC), or expanded cells thereof, for use in treating or preventing macular degeneration or diabetic retinopathy.

6. Adult STRO-1^{bright} mesenchymal precursor cells (MPC), or expanded cells thereof, according to claim 5, wherein the macular degeneration is dry age-related macular degeneration or wet age-related macular degeneration.

7. Adult STRO-1^{bright} mesenchymal precursor cells (MPC), or expanded cells thereof, according to claim 5 or claim 6, wherein the stem cells are obtained from bone marrow.

8. Adult STRO-1^{bright} mesenchymal precursor cells (MPC), or expanded cells thereof, according to any one of claims 5 to 7, wherein at least some of the cells are genetically modified.

## Patentansprüche

1. Verwendung von adulten mesenchymalen STRO-1 ^{bright}-Vorläuferzellen (MPC) oder daraus vermehrten Zellen für die Herstellung eines Medikaments zur Behandlung oder Verhütung einer Makuladegeneration oder einer diabetischen Retinopathie in einem Subjekt.

2. Verwendung nach Anspruch 1, wobei die Makuladegeneration eine altersbedingte trockene Makuladegeneration oder eine altersbedingte feuchte Makuladegeneration ist.

3. Verwendung nach Anspruch 1 oder Anspruch 2, wobei die Stammzellen aus Knochenmark gewonnen werden.

4. Verwendung nach einem der Ansprüche 1 bis 3, wobei zumindest einige von den Zellen genetisch modifiziert sind.

5. Adulte mesenchymale STRO-1 ^{bright}-Vorläuferzellen (MPC) oder daraus vermehrte Zellen zur Verwendung bei der Behandlung oder Verhütung einer Makuladegeneration oder einer diabetischen Retinopathie.

6. Adulte mesenchymale STRO-1^{bright}-Vorläuferzellen (MPC) oder daraus vermehrte Zellen nach Anspruch 5, wobei die Makuladegeneration eine altersbedingte trockene Makuladegeneration oder eine altersbedingte feuchte Makuladegeneration ist.

7. Adulte mesenchymale STRO-1 ^{bright}-Vorläuferzellen (MPC) oder daraus vermehrte Zellen nach Anspruch 5 oder Anspruch 6, wobei die Stammzellen aus Knochenmark gewonnen werden.

8. Adulte mesenchymale STRO-1^{bright}-Vorläuferzellen (MPC) oder daraus vermehrte Zellen nach einem der Ansprüche 5 bis 7, wobei zumindest einige von den Zellen genetisch modifiziert sind.

## Revendications

1. L'utilisation de cellules précurseurs mésenchymateuses (MPC) adultes STRO-1^{bright}, ou de cellules expansées de celles-ci, dans la fabrication d'un médicament destiné au traitement ou à la prévention de la dégénération maculaire ou de la rétinopathie diabétique chez un sujet.

2. L'utilisation selon la Revendication 1, où la dégénération maculaire est une dégénération maculaire liée à l'âge sèche ou une dégénération maculaire liée à l'âge humide.

3. L'utilisation selon la Revendication 1 ou 2, où les cellules souches sont obtenues à partir de moelle osseuse.

4. L'utilisation selon l'une quelconque des Revendications 1 à 3, où au moins certaines des cellules sont génétiquement modifiées.

5. Les cellules précurseurs mésenchymateuses (MPC) adultes STRO-1^{bright}, ou des cellules expansées de celles-ci, destinées au traitement ou à la prévention de la dégénération maculaire ou de la rétinopathie diabétique.

6. Les cellules précurseurs mésenchymateuses (MPC) adultes STRO-1^{bright}, ou des cellules expansées de celles-ci, selon la Revendication 5, où la dégénération maculaire est une dégénération maculaire liée à l'âge sèche ou une dégénération maculaire liée à l'âge humide.

7. Les cellules précurseurs mésenchymateuses (MPC) adultes STRO-1^{bright}, ou des cellules expansées de celles-ci, selon la Revendication 5 ou 6, où les cellules souches sont obtenues à partir de moelle osseuse.

8. Les cellules précurseurs mésenchymateuses (MPC) adultes STRO-1^{bright}, ou des cellules expansées de celles-ci, selon l'une quelconque des Revendications 5 à 7, où au moins certaines des cellules sont génétiquement modifiées.
